(19) Europäisches Patentamt
European Patent Office
Office européen des brevets

(11) Publication number: **0 170 121 B1**

(12) # EUROPEAN PATENT SPECIFICATION

(45) Date of publication of patent specification: **19.06.91**

(51) Int. Cl.5: **C07C 217/04**, C07C 229/38, C07C 311/16, C07D 307/80, A61K 31/135, A61K 31/34

(21) Application number: **85108609.0**

(22) Date of filing: **10.07.85**

(54) **2-Hydroxy-3-aryloxypropyl or 2-hydroxy-2-benzofuranyl ethyl tertiary amine having an anti-hyperglycaemic and/or anti-obesity activity.**

(30) Priority: **19.07.84 GB 8418472**

(43) Date of publication of application:
**05.02.86 Bulletin 86/06**

(45) Publication of the grant of the patent:
**19.06.91 Bulletin 91/25**

(84) Designated Contracting States:
**AT BE CH DE FR GB IT LI LU NL SE**

(56) References cited:
**EP-A- 0 099 707**
**EP-A- 0 101 069**
**EP-A- 0 140 243**
**US-A- 4 146 638**

(73) Proprietor: **BEECHAM GROUP PLC**
**Beecham House Great West Road**
**Brentford Middlesex TW8 9BD(GB)**

(72) Inventor: **Ainsworth, Anthony Trevor**
**3 Crescent Road**
**Bishop's Stortford Hertfordshire(GB)**
Inventor: **Cawthorne, Michael Anthony**
**16 Millais**
**Horsham Sussex(GB)**

(74) Representative: **Rutter, Keith et al**
**Beecham Pharmaceuticals Great Burgh Yew Tree Bottom Road**
**Epsom Surrey KT18 5XQ(GB)**

EP 0 170 121 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The invention relates to tertiary amines which have anti-hyperglycaemic and/or anti-obesity activity, to processes for their preparation; pharmaceutical compositions containing them and their use in agriculture.

Tertiary amines having anti-hyperglycaemic and/or anti-obesity activity are already known. For example, EP 101069 A discloses certain phenylethanolamine derivatives with anti-obesity and hypoglycaemic activity; the nitrogen atom of these is substituted by a 2-hydroxyphenethyl radical, by one phenalkyl or thienylalkyl radical, and by a further 2-hydroxyphenethyl radical or by a 2-hydroxyalkyl radical.

EP 140243 A also discloses certain tertiary amines having anti-hyperglycaemic and/or anti-obesity activity. The disclosures of EP 140243 A are relevant to the invention only in so far as is dictated by Article 54(3) of the European Patent Convention.

We have now discovered a group of pharmacologically active tertiary amines which are aryloxypropanolamine derivatives, the hydroxy group being on the carbon atom $\beta$ to the nitrogen atom. The compounds have potent anti-obesity and anti-hyperglycaemic activity coupled with low cardiac stimulation activity.

Accordingly, the present invention provides a compound of the general formula (I):

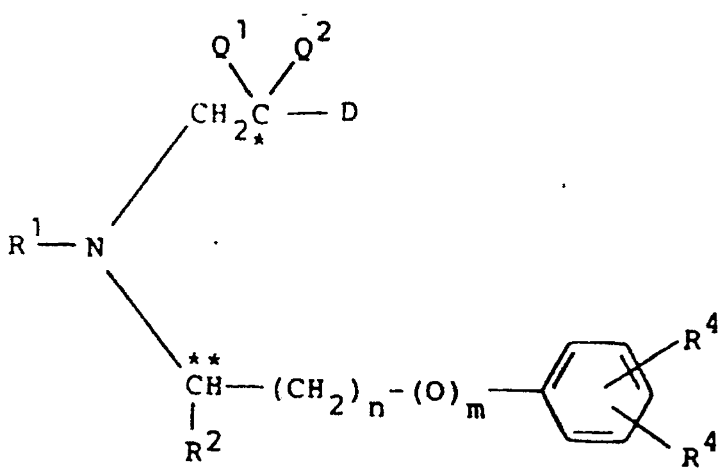

(I)

or a pharmaceutically acceptable salt thereof,
in which
$R^1$
represents a 2-hydroxy-2-(benzofuran-2-yl)ethyl group or a radical of the general formula (a) or (b):

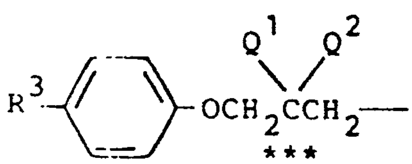

or

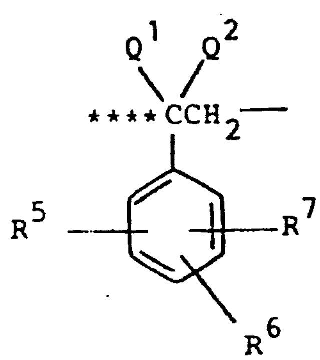

(a) (b)

$R^2$
represents a hydrogen atom or a methyl group,

D

represents either a benzofuran-2-yl moiety or a radical of general formula (c):

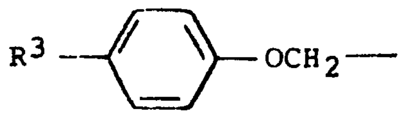

(c)

$R^3$

represents a hydrogen atom or a $CH_2CONH_2$ group, and where there are two $R^3$s in the compound these are the same or different, provided that there is a maximum of one $CH_2CONH_2$ group in the compound,

$R^4$

represents a radical $-(CH_2)_aCOOH$, $-O(CH_2)_bCOOH$, or $-C(R^8)=C(R^9)COOH$ or an ester or an amide, lower alkylamide, di-lower alkylamide or saturated 5- or 6-membered ring amide derivative of one of these groups, or $-SO_2NR^{10}R^{11}$, $-(CH_2)_aCH_2X$, or $-O(CH_2)_bCH_2X$

in which

a

represents zero or an integer from 1 to 6,

b

represents an integer from 1 to 6,

$R^8$ and $R^9$,

which may be the same or different, each represents a hydrogen atom or a methyl group,

$R^{10}$ and $R^{11}$,

which may be the same or different, each represents a hydrogen atom or a lower alkyl radical or, together with the nitrogen atom to which they are attached, form a saturated 5- or 6- membered ring, and

x

represents a hydrogen atom or a lower alkyl radical, a hydroxy group or an ester thereof,a lower alkoxy radical, an unsubstituted or substituted benzyloxy radical or a (lower alkyl)carbonyl radical, or a radical $NR^{10}R^{11}$ in which $R^{10}$ and $R^{11}$ have the meanings given above,

$R^{4'}$

represents a hydrogen or a halogen atom or a lower alkyl or lower alkoxy radical, a $CF_3$ group or a hydroxy group or an ester thereof,

$R^5$

represents a hydrogen or halogen atom, a hydroxy or hydroxymethyl group or an ester of either of these groups, or a group $CF_3$, $NH_2$, $NHR^{12}$, $NHCOOR^{12}$, or $CH_2SO_2R^{12}$ in which $R^{12}$ represents a lower alkyl radical,

$R^6$

represents a hydrogen or halogen atom, or a hydroxy group or an ester thereof,

$R^7$

represents a hydrogen or halogen atom,

n

represents 1 or 2,

m

represents 0 or 1,

provided that n + m = 1 or 2, and

$Q^1$

represents a hydrogen atom and

$Q^2$ represents a hydroxy group or a pharmaceutically acceptable in-vivo hydrolysable ester thereof, or $Q^1$ and $Q^2$ together represent an oxo group,the two $Q^1Q^2$ pairs in the compound being the same or different, provided there is a maximum of one $Q^1Q^2$ oxo group in the compound;

with the proviso that when $R^1$ represents a radical of the abovementioned formula (a), wherein $R^3$ and $Q^1$ each represent a hydrogen atom and $Q^2$ represents a hydroxy group or a pharmaceutically acceptable in-vivo hydrolysable ester thereof, or $R^1$ represents a radical of the abovementioned formula (b) wherein $R^5$ represents a hydrogen or halogen atom or a trifluoromethyl group and $R^6$, $R^7$ and $Q^1$ each represent a hydrogen atom and $Q^2$ represents a hydroxy group or an in-vivo hydrolysable ester thereof; and $R^2$ represents a hydrogen atom or a methyl group; and

D represents a radical of the abovementioned formula (c) wherein $R^3$ represents a hydrogen atom; $Q^1$ represents a hydrogen atom and $Q^2$ represents a hydroxy group or an in-vivo hydrolysable ester thereof;
$R^4$ represents a para substituent on the phenyl ring, relative to the $-(O)_m-$ moiety, and represents either a radical $-(CH_2)_aCO_2H$, $-C(R^8)=C(R^9)CO_2H$, or an ester or amide, lower alkylamide or di- lower alkylamide of one of these groups; or $-SO_2NR^{10}R^{11}$; $-(CH_2)_aCH_2X$, wherein X represents hydroxy or a (lower alkyl)-carbonyl radical;
$-O(CH_2)_bCH_2X$ wherein X represents hydroxy, lower alkoxy or an unsubstituted benzyloxy group;
and a represents zero or an integer from 1 to 6;
and b represents an integer from 1 to 6;
and $R^8$ represents a hydrogen atom or a methyl group;
and $R^9$, $R^{10}$ and $R^{11}$ each represents a hydrogen atom;
and n represents 1 or 2 and m represents zero; then $R^{4'}$ does not represent a hydrogen atom.

The compounds of the general formula (I) have, depending on the meaning of $Q^1$, $Q^2$ and $R^2$, up to three asymmetric carbon atoms, marked with asterisks in the formula. These compounds may, therefore, exist in up to eight stereoisomeric forms. Also, when $R^4$ represents a $-C(R^8)=C(R^9)COOH$ radical, geometrical isomers are possible. The present invention encompasses all stereoisomers and geometrical isomers of the compounds of the general formula (I) whether free from other isomers or admixed with other isomers in any proportion, and thus includes, for instance, racemic mixtures of enantiomers.

When used herein in connection with alkyl and alkoxy radicals, or with alkanols, the term "lower" indicates that the radical or compound has from 1 to 6 carbon atoms, preferably 1 to 4 carbon atoms. The alkyl radical or the alkyl moiety in the alkoxy radical or alkanol compound may be straight- or branched-chain, e.g. a methyl, ethyl, n-propyl, isopropyl, n-butyl, isobutyl or a tert.-butyl group.

The radical $Q^1$ shown in the general formula (I) is preferably a hydrogen atom, and $Q^2$ preferably a hydroxy group or ester thereof. Similarly, in each of the radicals (a) and (b) represented by $R^1$ preferably $Q^1$ represents a hydrogen atom and $Q^2$ represents a hydroxy group or a pharmaceutically acceptable in-vivo hydrolysable ester thereof. Suitably in-vivo hydrolysable esters of hydroxy groups are those used conventionally in the art, preferably they are esters of lower alkyl carboxylic acids. Preferably each radical represented by $Q^2$ is a free hydroxy group.

Thus, there should especially be mentioned compounds of the general formula (I)':

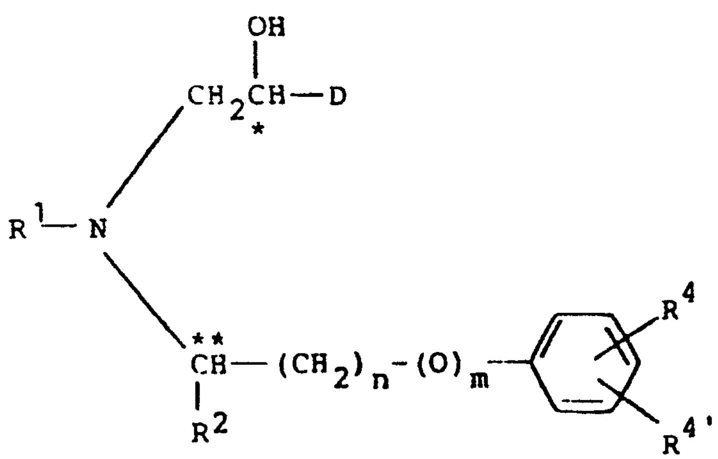

(I')

or a pharmaceutically acceptable salt thereof,
in which $R^1$ represents a 2-hydroxy-2-(benzofuran-2-yl) ethyl group or a radical of the general formula (a') or (b'):

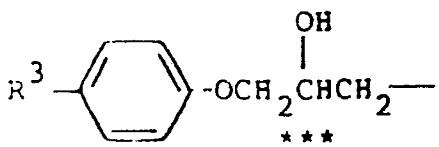 or 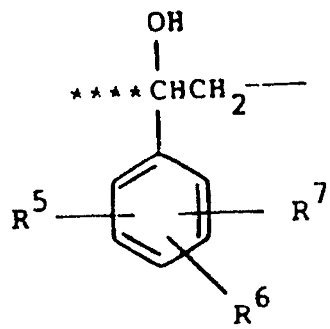

(a′) (b′)

and $R^2$,$R^3$,$R^4$,$R^{4'}$,$R^5$,$R^6$,$R^7$, m and n are as defined in relation to formula (I); and D represents a benzofuran-2-yl moiety or a radical of formula (c) as defined in relation to formula (I).

More especially, there should be mentioned compounds of the general formula (I) or (I') in which m represents zero.

Preferably $R^2$ in general formulae (I) or (I') represents a methyl group.

Preferably in the moiety or each moiety of the formula

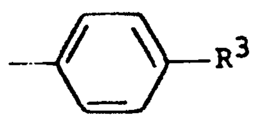

$R^3$ represents a hydrogen atom.

Preferably, in the moiety of the formula

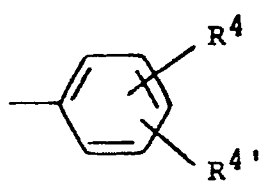

$R^4$ is in the para position. Preferably also $R^{4'}$ represents a hydrogen atom. Thus, especially, there should be mentioned compounds of the general formula (I') in which the moiety

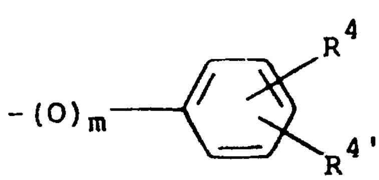

is

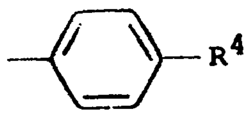

In each of the general formulae (I) and (I'), $R^4$ may represent, for example, a radical of the formula $-(CH_2)_aCOOH$, or an ester or amide derivative thereof mentioned above. Preferably a represents zero.

$R^4$ may also represent, for example, a radical of the formula $-O(CH_2)_bCOOH$ or an ester or amide derivative thereof mentioned above. Preferably b represents 1.

$R^4$ may further represent, for example, a radical of the formula $-C(R^8) = (R^9)COOH$ or an ester or amide derivative thereof mentioned above. Each of $R^8$ and $R^9$ preferably represents a hydrogen atom.

An ester derivative of one of the carboxy- containing $R^4$ groups is, for example, a lower alkyl ester, e.g. a methyl or ethyl ester.

An amide derivative of one of the carboxy containing $R^4$ groups is a $CONR^{10}R^{11}$ group in which $R^{10}$ and $R^{11}$ have the meanings given above.

$R^4$ may furthermore represent a radical of the formula $-SO_2NR^{10}R^{11}$, $-(CH_2)_aCH_2NR^{10}R^{11}$ or $-O(CH_2)_bCH_2NR^{10}R^{11}$.

In the various radicals mentioned above, each of $R^{10}$ and $R^{11}$ may represent a lower alkyl radical, especially a methyl group, or a hydrogen atom. When $NR^{10}R^{11}$ forms a saturated 5- or 6-membered ring this may, if desired, contain a further hetero atom. Preferably, $NR^{10}R^{11}$ represents a 1-pyrrolidinyl or 1-piperidyl group, or, especially, in the case of an $-O(CH_2)_bCH_2NR^{10}R^{11}$ radical, a 1-piperazinyl or a 4-morpholinyl radical.

Other possibilities for $R^4$ are other radicals of the formulae $-(CH_2)_aCH_2X$ or $-O(CH_2)_bCH_2X$, preferably $CH_2X$ where X has the meaning given above, or $-O(CH_2)_bCH_2OH$, especially $-O(CH_2)_2OH$.

Preferably, however, $R^4$ represents a carboxy-containing radical or an ester or amide derivative thereof.

More preferably, $R^4$ represents a carboxy group or a radical $-OCH_2CO_2H$ or an ester or amide thereof. Preferred esters are the methyl and ethyl esters.

$R^1$ may represent a 2-hydroxy-2-(benzofuran-2-yl)ethyl group or a moiety of the formula

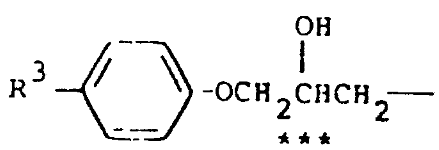

or

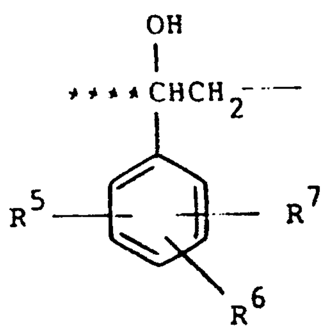

(a')

(b')

wherein $R^3$, $R^6$ and $R^7$ are as defined in relation to formula (I) and $R^5$ represents a hydrogen or halogen atom, a hydroxy or hydroxymethyl group or an ester of either of these groups, or a group $NH_2$ or $NHR^{12}$ in which $R^{12}$ represents a lower alkyl radical.

When there are two of the radicals (a') in the molecule, these may be the same or different, provided that at least one $R^3$ represents a hydrogen atom.

In the radical of the formula (b'), a halogen atom represented by $R^6$ and/or $R^7$ is preferably a chlorine atom, and in the radical $R^5$ a lower alkyl radical $R^{12}$ is preferably a methyl group.

An ester of a hydroxy or hydroxy-containing group represented by $R^5$ or $R^6$ is an ester of, for example,

an aromatic or lower alkyl carboxylic acid.

Preferably, however, the moiety of the formula (b) is one of the following groups:

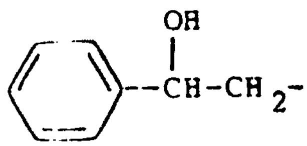
,
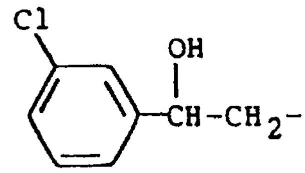

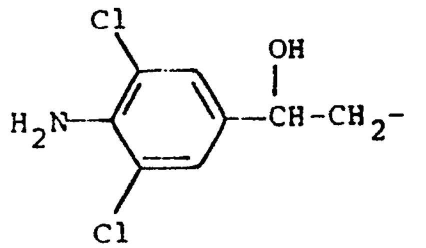
,
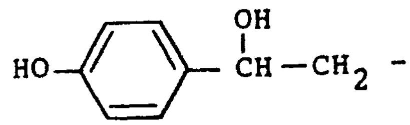

In a particularly preferred aspect the present invention provides a compound selected from the group consisting of:

methyl 4-[(R)-2-[bis-[(S)-2-hydroxy-3-phenoxypropyl]amino]propyl]benzoate;

methyl 4-[(R)-2-[bis-[(R)-2-hydroxy-3-phenoxypropyl]amino]propyl]benzoate;

N-methyl 4-[(R)-2-[bis-[(S)-2-hydroxy-3-phenoxypropyl]amino]propyl]benzamide;

methyl 4-[(R)-2-[N-[(R)-2-hydroxy-2-phenethyl] -N-[(S)-2-hydroxy-3-phenoxypropyl]amino]propyl] benzoate;

methyl 4-[(R)-2-[N-[(R)-2-hydroxy-2-phenethyl] -N-[(R)-2-hydroxy-3-phenoxypropyl]amino]propyl] benzoate;

N-methyl 4-[(R)-2-[N'-[(R)-2-hydroxy -2-phenethyl]-N'-[(S)-2-hydroxy-3-phenoxypropyl] amino]propyl]-benzamide;

N-methyl 4-[(R)-2-[N'-[(R)-2-hydroxy-2-phenethyl]-N'-[(R)-2-hydroxy-3-phenoxypropyl] amino]propyl] benzamide;

4-[2-[N-[2-(3-chlorophenyl)-2-hydroxyethyl]-N-[2-hydroxy-3-phenoxypropyl]amino]propyl] phenoxyacetamide;

methyl 4-[2-[N-[2-hydroxy-2-[4-hydroxy-3-hydroxymethylphenyl]ethyl]-N-[2-hydroxy-3-phenoxypropyl]-amino]propyl]benzoate;

methyl 4-[(R)-2-[N-[2-[4-amino-3,5-dichlorophenyl]-2-hydroxyethyl]-N-[(S)-2-hydroxy-3-phenoxypropyl]-amino]propyl]benzoate;

methyl 4-[2-[N-[2-(3-chlorophenyl)-2-hydroxyethyl]-N-[2-hydroxy-3-(4-carboxamidomethyl) phenoxypropyl]-amino]propyl]phenoxyacetate;

4-[(R)-3-[N-(R)-2-hydroxy-2-phenethyl-N-[(S)-2-hydroxy-3-phenoxypropyl]amino]butyl]benzamide;

4-[(R)-2-[bis-[(S)-2-hydroxy-3-phenoxypropyl] amino]propyl]phenoxyacetamide;

ethyl 4-[(R)-2-[N-[(R)-2-hydroxy-2-phenethyl]-N-[(S)-2-hydroxy-3-phenoxypropyl]amino]propyl] phenoxyacetate;

4-[(R)-2-[N-[(R)-2-hydroxy-2-phenethyl]-N-[(S) -2-hydroxy-3-phenoxypropyl]amino]propyl] phenoxyacetamide;

ethyl 4-[(R)-2-bis-[(S)-2-hydroxy-3-phenoxypropyl] amino]propyl]phenoxyacetate;

ethyl 4-[(R)-2-[N-[(R)-2-(3-chlorophenyl) -2-hydroxyethyl]-N-[(S)-2-hydroxy-3-phenoxypropyl]amino]propyl] phenoxyacetate;

4-[(R)-2-[N-[(R)-2-(3-chlorophenyl) -2-hydroxyethyl]-N-[(S)-2-hydroxy-3-phenoxypropyl]amino] propyl]-phenoxyacetamide;

4-[(R)-2-[N-[(R)-2-hydroxy-2-phenethyl]-N-[(S)-2-hydroxy-3-phenoxypropyl]amino]propyl] phenoxyethylamine;

ethyl 4-[(R)-3-[N-[(R)-2-hydroxy-2-phenethyl] -N-[(S)-2-hydroxy-3-phenoxypropyl]amino]butyl] phenoxyacetate;

4-[(R)-2-[N-[(R)-2-hydroxy-2-phenethyl] -N-[(S)-2- hydroxy-3-phenoxypropyl]amino]propyl] phenoxyethanol;

ethyl 4-[(R)-2-[N-[(R)-2-hydroxy-2-phenethyl] -N-[(R)-2-hydroxy-3-phenoxypropyl]amino]propyl] phenoxyacetate;

methyl 4-[(R)-2-[N-[(R)-2-hydroxy-2-phenethyl] -N-[(S)-2-hydroxy-3-phenoxypropyl]amino]propyl] phenoxyacetate;

4-[(R)-2-[N-[(R)-2-hydroxy-2-phenethyl] -N-[(S)-2-hydroxy-3-phenoxypropyl]amino] propoxy] phenoxyacetamide;
ethyl 4-[(R)-2-[N-[2-(2-benzofuranyl) -2-hydroxyethyl]-N-[(S)-2-hydroxy-3-phenoxypropyl] amino]propyl]-phenoxyacetate
ethyl 4-[(R)-2-[N-[(R)-2-hydroxy-2-phenethyl] -N-[2-(2-benzofuranyl)-2-hydroxyethyl]amino] propyl] phenoxyacetate;
4-[(R)-2-[N-[(R)-2-hydroxy-2-(4-hydroxyphenyl) ethyl]-N-[(R)-2-hydroxy-3-phenoxypropyl] amino]propyl]-phenoxyacetamide;
methyl 4-[2-[N-[2-(4-amino-3,5-dichlorophenyl) -2- hydroxy-ethyl]-N-[2-hydroxy-3-phenoxypropyl] amino] propyl]phenoxyacetate; and
4-[2-[N-[2-(4-amino-3,5-dichlorophenyl) -2-hydroxyethyl]-N-[2-hydroxy-3-phenoxypropyl] amino]propyl] phenoxyacetamide; or a pharmaceutically acceptable salt thereof.

The absolute configuration of any compound of the general formula (I) may be determined by conventional x-ray crystallographic techniques.

Suitably, the "**" asymmetric carbon has the R-configuration.

Suitably the "****" asymmetric carbon has the R-configuration.

When $R^1$ is a radical of formula (a) and $R_2$ is methyl and $Q^1$ is hydrogen, a favoured enantiomer is that wherein the asymmetic carbons have the following configurations:

''*'' = S; ''**'' = R; and ''***'' = S; or
''*'' = S; ''**'' = R; and ''***'' = R; or
''*'' = R; ''**'' = R; and ''***'' = R; or
''*'' = R; ''**'' = R; and ''***'' = S.

When $R^1$ is a radical of formula (b) and $R^2$ is methyl and $Q^1$is hydrogen, a favoured enantiomer is that wherein the asymmetric carbons have the following configurations:

''*'' = S; ''**'' = R; and ''****'' = R; or
''*'' = R; ''**'' = R; and ''****'' = R.

The pharmaceutically acceptable salts of compounds of the general formula (I) may be acid addition salts; salt formation is possible for example with the nitrogen atom shown in the formula (I). Salts may also be formed, for example, with carboxy groups. Internal salts should also be mentioned. The hydrates of the pharmaceutically acceptable salts are also encompassed in the present invention.

Acid addition salts may be, for example, salts with inorganic acids such, for example, as hydrochloric acid, hydrobromic acid, orthophosphoric acid or sulphuric acid, or organic acids such, for example, as methanesulphonic acid, toluenesulphonic acid, acetic acid, propionic acid, lactic acid, citric acid, fumaric acid, malic acid, succinic acid, salicylic acid or acetylsalicylic acid.

Salts formed with carboxy groups may be, for example alkali metal, e.g. sodium or potassium, or ammonium salts or alkaline earth metal salts, e.g. calcium or magnesium salts.

The invention also provides a process A for the preparation of a compound of the general formula (I) or a pharmaceutically acceptable salt thereof, which comprises reacting a compound of the general formula (II):

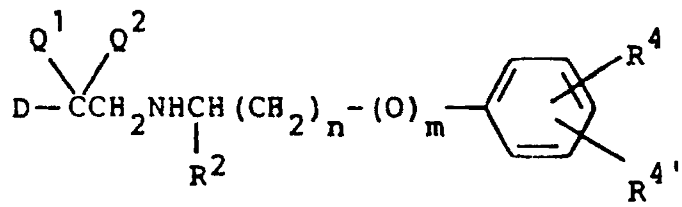

(II)

in which $R^2$, D, $R^4$, $R^{4'}$, n, m, $Q^1$ and $Q^2$ are as defined in relation to formula (I) with
(i) a compound of the general formula

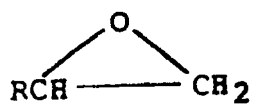

(III)

in which R represents a benzofuran-2-yl moiety or a group of the general formula

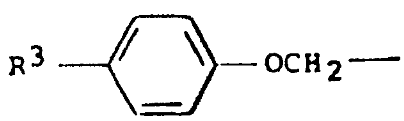

or

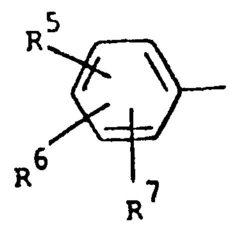

in which $R^3$, $R^5$, $R^6$ and $R^7$ are as defined in relation to formula (I), or
(ii) a compound of the general formula (IV):

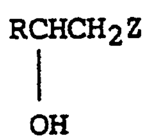

(IV)

in which R is as defined in relation to formula (III) and z represents a leaving group, or an ester thereof, and, if desired, converting a resulting compound of the general formula (I) into another compound of the general formula (I) and/or preparing a pharmaceutically acceptable salt thereof.

The present invention also provides a process B for the preparation of a compound of the general formula (I) in which there is one oxo group represented by $Q^1 + Q^2$, or a pharmaceuticaly acceptable salt thereof, which comprises reacting a compound of the general formula (II), provided that $Q^1$ and $Q^2$ do not represent an oxo group, with a compound of the general formula

$RCOCH_2Z$ (v)

in which R and Z are as defined in relation to formula (III) and, if desired, converting the resulting compound of the general formula (VI):

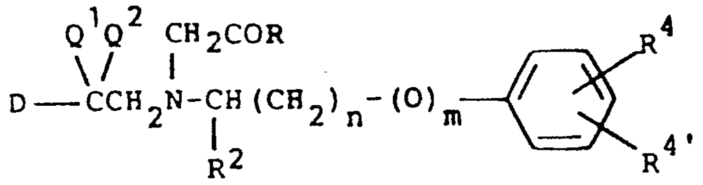

(VI)

in which R, $R^2$, D, $R^4$, $R^{4'}$, n, m, $Q^1$ and $Q^2$ are as defined in relation to formula (I) and provided that $Q^1$ and $Q^2$ do not represent an oxo group, into another compound of the general formula (VI) (or I), or into a pharmaceutically acceptable salt thereof.

A leaving group Z is any group that will, under the reaction conditions, cleave from the starting material thus promoting reaction at a specified site. Suitable examples of such groups are halogen atoms, mesyloxy groups and tosyloxy groups. Preferably in compounds of formula (C) and (IV) Z represents a tosyloxy group, and in compounds of formula (D) and (V) Z represents a bromine atom.

A starting material of the general formula (II) may be prepared, for example, by reacting a compound of the general formula (VII)a or (VII)b:

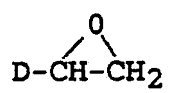

$D-CO.CH_2Z$

(VII)a (VII)b

in which D is as defined in relation to formula (I), and Z is as defined in relation to formula (V), with a compound of the general formula (VIII):

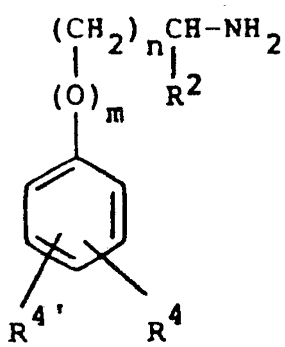

(VIII)

in which $R^2$, $R^4$, $R^{4'}$, n and m are as defined in relation to formula (I).

Compounds of formulae (III), (IV), (V), (VII), (a and b), and (VIII) are either known compounds or can be prepared from known compounds by known processes or processes analogous to known processes.

The invention also provides a process C for the preparation of a compound of the general formula (I) or a pharmaceutically acceptable salt thereof, which comprises reacting a compound of the general formula (IX):

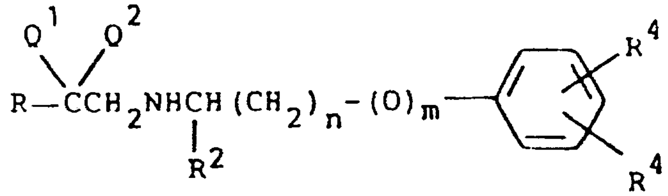

(IX)

in which $R^2$, $R^4$, $R^{4'}$, n, m, $Q^1$ and $Q^2$ are as defined in relation to formula (I) and R is as defined in relation to formula (III), with the hereinbefore defined compound of the general formula (VII)a or (VII)b providing that if $Q^1$ and $Q^2$ together represent a carbonyl group then compound (IX) is reacted with compound (VII)a and, if desired, converting a resulting compound of the general formula (I) into another compound of the general formula (I) and/or into a pharmaceutically acceptable salt thereof.

Starting materials of the general formula (IX) are described, for example, in EP 0023385 and may be prepared, for example, by reacting a compound of the hereinbefore defined general formulae (III) and (VIII) or (V) and (VIII).

The reaction between compounds of the general formulae (II) and (III), (III) and (VIII), (VII)a and (VIII), (IX) and (VII)a, or (X) and (VIII) is advantageously carried out in a protic solvent, e.g. a lower alkanol having at least 2 carbon atoms, at reflux, preferably in ethanol.

Reaction between compounds of the general formulae (II) and (IV) is advantageously carried out in dimethyl sulphoxide, for example at a temperature in the range of from 30 to 80°C, e.g. about 50°C and advantageously for a period of time of 1 to 4 days, e.g. about 3 days.

The reaction between the compounds of the general formulae (II) and (V) or (V) and (VIII) is advantageously carried out in butanone or acetonitrile at reflux, if desired in the presence of a base.

The invention also provides a process D for the preparation of a compound of the general formula (I) or a pharmaceutically acceptable salt thereof, which comprises reacting a compound of the general formula (II):

(i) with a compound of the general formula (X):

R-CO CHO (X)

in which R, is as defined in relation to formula (III) and subsequently treating with a reducing agent, or

(ii) with a compound of the general formula (XI):

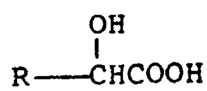

(XI)

in which R, is as defined in relation to formula (III), and subsequently reducing the resulting hydroxyamide, and, if desired, converting a resulting compound of the general formula (I) into another compound of the general formula (I) and/or into a pharmaceutically acceptable salt thereof.

In method (i) above, the reduction may be carried out, for example, with sodium cyanoborohydride; in method (ii), the reaction of compounds of the general formulae (II) and (XI) may be carried out in the presence, for example, of dicyclohexyl carbodiimide or other suitable condensing agent, and the subsequent reduction may be carried out, for example, with lithium aluminium hydride or a borane reducing agent, for example borane methyl sulphide complex.

The invention also provides a process (E) for the preparation of a compound of the general formula (I) or a pharmaceutically acceptable salt thereof, which comprises reacting a compound of the general formula (XII):

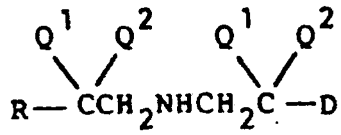

(XII)

in which D, $Q^1$ and $Q^2$ are as defined in relation to formula (I) and R is as defined in relation to formula (III),

(i) with a compound of the general formula (XIII):

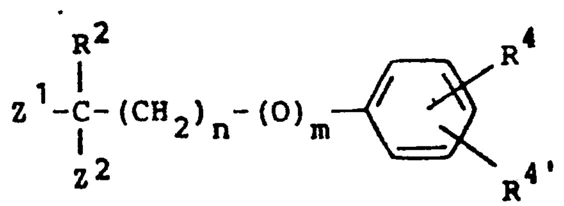

(XIII)

in which $R^2$, $R^4$, $R^{4'}$, n and m are as defined in relation to formula (I) and $Z^1$ represents a leaving group and $Z^2$ represents a hydrogen atom, or

(ii) with a compound of the general formula (XIII) in which $R^2$, $R^4$, $R^{4'}$, n and m are as defined in relation to formula (I) and $Z^1$ and $Z^2$ together represent an oxo group;

and if necessary treating with a reducing agent, and, if desired, converting a resulting compound of the general formula (I) into another compound of the general formula (I) and/or into a pharmaceutically acceptable salt thereof.

Reaction of compounds of the general formulae (XII) and (XIII) is advantageously carried out, in the case where $Z^1$ represents a leaving group, for example one of those mentioned above, more especially a tosyloxy group or bromine atom, in dimethyl sulphoxide or methanol, for example at a suitable temperature in the range of from 30 to 80 °C, e.g. about 50 °C and advantageously for a period of time of 1 to 4 days, e.g. about 3 days. In the case where $Z^1$and $Z^2$ together represent an oxo group, the subsequent reduction is, for example, with sodium cyanoborohydride.

A compound of the general formula (I) may, if desired, be converted into another compound of the general formula (I) by a method known per se. For example, an acid may be converted into an ester, an ester may be converted into an acid, amide or alcohol, or an alcohol into an ester thereof, or a phenoxyacetate converted into a phenoxyacetamide and the phenoxyamide optionally further converted into the phenoxyethylamine.

Also, for example, a compound of the general formula (VI) or a salt thereof may be reduced to form a compound of the general formula (I).

Thus, the invention also provides for a process F for the preparation of a compound of the general formula (I) or a pharmaceutically acceptable salt thereof, which comprises reducing a compound of the general formula (VI) in which R, $R^2$, D, $R^4$, $R^{4'}$, n, m, $Q^1$ and $Q^2$ are as defined in relation to formula (I), or a salt thereof, and, if desired, converting a resulting compound of the general formula (I) into another compound of the general formula (I) and/or into a pharmaceutically acceptable salt thereof.

The reduction of the compound of the general formula (VI) is advantageously carried out with sodium borohydride and preferably in a lower alkanol.

The salts of compounds of the general formula (I) may be produced, for example, by treating a compound of general formula (I) with the appropriate acid or base.

Compounds of the general formula (I) and pharmaceutically acceptable salts thereof, produced by the above processes, may be recovered by conventional methods.

Compounds of the general formula (I) may be separated into diastereoisomeric pairs of enantiomers by, for example, fractional crystallisation from a suitable solvent, for example methanol or ethyl acetate or a mixture thereof. The pair of enantiomers thus obtained may be separated into individual stereoisomers by conventional means, for example by the use of an optically active acid as a resolving agent.

Suitable optically active acids which may be used as resolving agents are described in 'Topics in Stereochemistry', Vol. 6, Wiley Interscience, 1971, Allinger, N.L. and Eliel, W.L. Eds.

Alternatively, any enantiomer of a compound of the general formula (I) may be obtained by stereospecific synthesis using optically pure starting materials of known configuration.

For example, reaction of one enantiomer of a compound of the general formula (VII)a or (III) with one enantiomer of a compound of the general formula (VIII) gives a compound of a general formula (II) or (IX) respectively as a single enantiomer. The enantiomer may then be further reacted with a single enantiomer, for example the enantiomer of compound of the general formula (II) with one enantiomer of a compound of the general formula (III) or the enantiomer of compound of the general formula (IX) with one enantiomer of a compound of the general formula (VII)a, to produce a single enantiomer of the general formula (I).

As previously indicated, the compounds of the present invention have valuable pharmacological properties.

The present invention also provides a compound of the general formula (I) or a pharmaceutically acceptable salt thereof for use as an active therapeutic substance.

In one aspect, the present invention provides a compound of the general formula (I) or a pharmaceutically acceptable salt thereof for use in the treatment of obesity in human or non-human animals.

The present invention further provides a compound of the general formula (I), or a pharmaceutically acceptable salt thereof, for use in the treatment of hyperglycaemia in human or non-human animals.

A compound of the general formula (I) or a pharmaceutically acceptable salt thereof may be administered per se or, preferably, as a pharmaceutical composition also comprising a pharmaceutically acceptable carrier.

Accordingly, the present invention also provides a pharmaceutical composition comprising a compound of the general formula (I) or a pharmaceutically acceptable salt thereof with a pharmaceutically acceptable carrier therefor.

As used herein the term "pharmaceutically acceptable" embraces compounds, compositions and ingredients for both human and veterinary use: for example the term "pharmaceutically acceptable salt" embraces a veterinarily acceptable salt.

The composition may, if desired, be in the form of a pack accompanied by written or printed instructions for use.

Usually the pharmaceutical compositions of the present invention will be adapted for oral administration, although compositions for administration by other routes, such as by injection, are also envisaged.

Particularly suitable compositions for oral administration are unit dosage forms such as tablets and capsules. Other fixed unit dosage forms, such as powders presented in sachets, may also be used.

In accordance with conventional pharmaceutical practice the carrier may comprise a diluent, filler, disintegrant, wetting agent, lubricant, colourant, flavourant or other conventional adjuvant.

Typical carriers include, for example, microcrystalline cellulose, starch, sodium starch glycollate, polyvinylpyrrolidone, magnesium stearate, sodium lauryl sulphate or sucrose.

Most suitably the composition will be formulated in unit dose form. Such unit dose will normally contain an amount of the active ingredient in the range of from 0.1 to 1000 mg, more usually 0.1 to 500 mg, and more especially 0.1 to 250 mg.

Conveniently, the active ingredient may be administered as a pharmaceutical composition hereinbefore defined, and this forms a particular aspect of the present invention.

In treating hyperglycaemic or obese humans the compound of the general formula (I) or pharmaceutically acceptable salt thereof may be taken in doses, such as those described above, one to six times a day in a manner such that the total daily dose for a 70 kg adult will generally be in the range of from 0.1 to 6000 mg, and more usually about 1 to 1500 mg.

In treating hyperglycaemic or obese non-human animals, especially dogs, the active ingredient may be administered by mouth, usually once or twice a day and in an amount in the range of from about 0.025 mg/kg to 25 mg/kg, for example 0.1 mg/kg to 20 mg/kg.

In a further aspect the present invention also provides a method for improving the weight gain and/or improving the feed utilisation efficiency and/or increasing lean body mass of livestock, which method comprises the administration to livestock of an effective non-toxic amount of a compound of formula (I) or a veterinarily acceptable salt thereof.

Whilst the compounds of formula (I) and the veterinarily acceptable salts thereof may be administered to any livestock in the abovementioned method, they are particularly suitable for improving the weight gain and/or feed utilisation efficiency and/or lean body mass in poultry, especially turkeys and chickens, cattle, pigs and sheep.

In the preceding method the compounds of formula (I) and veterinarily acceptable salts thereof will

normally be administered orally although non-oral modes of administration, for example injection or implantation, are also envisaged. Suitably the compounds are administered in the feed-stuff or drinking water provided for the livestock. Conveniently these are administered in the feed-stuff at from $10^{-3}$ ppm - 500ppm of total daily fed intake, more usually 0.01ppm to 250ppm, suitably less than 100ppm.

The particular formulations used will of course depend upon the mode of administration but will be those used conventionally in the mode of administration chosen.

For administration in feed-stuff the drugs are conveniently formulated as a premix in association with a suitable carrier.

Suitable carriers are inert conventional agents such as powdered starch. Other conventional feed-stuff premix carriers may also be employed.

No toxicological effects are indicated when a compound of formula (I) or a pharmaceutically acceptable salt thereof is administered in any of the abovementioned dosage ranges.

The following Examples illustrate the invention.

EXAMPLE 1

Methyl 4-[(R)-2-[bis-[(S)-2-hydroxy-3-phenoxypropyl]amino] propyl]benzoate

A mixture of methyl 4-[(R)-2-aminopropyl]benzoate (0.2 g) and (S)-phenoxymethyloxirane (0.28 g) was heated under reflux in ethanol for 43h. The solvent was evaporated and the residue chromatographed on alumina. Elution with chloroform gave methyl 4-[(R)-2-[bis-[(S)-2-hydroxy-3-phenoxypropyl]amino]propyl]-benzoate, m.p. 63-66 °C (ether-petrol), $[\alpha]_D^{25}$ (MeOH) = -13.5°.

$^1$H nmr ($CDCl_3$), ppm

1.09 (3H,d), 2.64-2.86 (5H,m), 2.89-2.97 (1H,m), 3.11 (2H, disappears on $D_2O$), 3.13-3.21 (1H,m), 3.90 (3H,s), 3.94 (6H,s), 6.87-7.01 (6H,m), 7.26-7.33 (6H,m), 7.97 (2H,d).

$^{13}$C nmr ($d_6$-DMSO), ppm

15.31, 51.89, 53.74, 58.35, 67.75, 70.16, 114.32, 120.32, 127.09, 129.01, 129.35, 129.44, 146.57, 158.64, 166.17.

EXAMPLE 2

Methyl 4-[(R)-2-[bis-[(R)-2-hydroxy-3-phenoxypropyl]amino] propyl]benzoate, fumarate

A mixture of methyl 4-[(R)-2-aminopropyl]benzoate (0.16 g) and (R)-phenoxymethyloxirane (0.224 g) was heated under reflux in ethanol for 76h. The solvent was evaporated and the residue chromatographed on kieselgel. Elution with chloroform gave an oil which was converted to methyl 4-[(R)-2-[bis-[(R)-2-hydroxy-3-phenoxypropyl]amino]propyl] benzoate, fumarate, m.p. 44-50 °C.

$^1$H nmr ($d_6$-DMSO), ppm

0.97 (3H,d), 2.44-2.81 (6H,m), 3.15 (1H,q), 3.2-3.5 (2H, broad, disappears with $D_2O$), 3.55-3:9 (6H,m), 3.76 (3H,s), 6.63 (2H,s), 6.73 (4H,d), 6.88 (2H,t), 7.16-7.27 (6H,m), 7.71 (2H,d).

$^{13}$C nmr ($d_6$-DMSO), ppm

13.54, 51.94, 52.78, 57.47, 67.47, 70.19, 114.27, 114.58, 120.35, 127.18, 129.01, 129.33, 129.40, 129.59, 134.07, 146.57, 158.65, 166.03, 166.15.

EXAMPLE 3

N-Methyl 4-[(R)-2-[biS-[(S)-2-hydroxy-3-phenoxypropyl] amino]propyl]benzamide, fumarate

The compound of Example 1 was dissolved in methanolic methylamine and heated in an autoclave at 100 °C for 20h. The solvent was evaporated and the residue chromatographed on kieselgel. Elution with methanol-chloroform (2:98) and treatment with fumaric acid gave N-methyl 4-[(R)-2-[bis-[(S)-2-hydroxy-3-phenoxypropyl]amino]propyl]benzamide, fumarate as a semi-solid.

$^1$H nmr ($d_6$-DMSO), ppm

0.85 (3H,d), 2.60-2.85 (5H,m), 2.77 (3H,d, collapses to singlet on $D_2O$), 2.95 (1H,m), 3.04 (1H,m), 3.84-3.95 (6H, m), 6.63 (2H,s), 6.86-6.93 (6H,m), 7.21-7.27 (6H,m), 7.72 (2H,d), 8.29 (1H, disappears with $D_2O$).

$^{13}$C nmr ($d_6$-DMSO), ppm

15.31, 26.16, 37.86, 53.75, 58.57, 67.53, 70.18, 114.45, 120.50, 127.07, 129.07, 129.51, 132.07, 134.07, 143.73, 158.71, 166.03, 166.63.

EXAMPLE 4

Methyl 4-[(R)-2-[N-[(R)-2-hydroxy-2-phenethyl)-N-[(S)-2-hydroxy-3-phenoxypropyl]amino]propyl]benzoate, hydrochloride, monohydrate

A mixture of methyl 4-[(R)-2-[N-[(R)-2-hydroxy-2-phenethyl] amino]propyl]benzoate (0.73 g) and (S)-phenoxymethyloxirane (0.35 g) was heated under reflux in ethanol for 48h. The solvent was evaporated and the residue chromatographed on kieselgel. Elution with chloroform gave an oil which was treated with ethereal hydrogen chloride to give methyl 4-[(R)-2-[N-[(R)-2-hydroxy-2-phenethyl]-N-[(S)-2-hydroxy-3-phenoxypropyl]amino]propyl]benzoate, hydrochloride, monohydrate, m.p. 90-95° C (ethylacetate/ether), $[\alpha]_D^{25}$ (MeOH) -96.3°.

$^1$H nmr ($d_6$-DMSO), ppm

1.21+1.24 (3H, two doublets), 3.01 (1H,t), 3.2-3.8 (4H,m), 3.85 (3H,s), 4.02-4.2 (4H,m), 4.56 (1H, broad), 5.28 (1H, broad), 6.19 (1H, disappears with $D_2O$), 6.40 (1H, disappears with $D_2O$), 6.98 (2H,d), 7.29-7.56 (10H,m), 7.93 (2H,d), 9.08+9.17 (1H, two broad singlets, disappear with $D_2O$).

$^{13}$C nmr ($d_6$-DMSO), free base, ppm

14.63, 51.87, 53.19, 58.04, 59.58, 67.53, 69.96, 70.99, 114.35, 120.29, 126.07, 126.74, 127.09, 127.79, 128.97, 129.33, 129.44, 144.11, 146.51, 158.61, 166.17.

EXAMPLE 5

Methyl 4-[(R)-2-[N-[(R)-2-hydroxy-2-phenethyl]-N-[(R)-2-hydroxy-3-phenoxypropyl]amino]propyl]benzoate hydrochloride

Methyl 4-[(R)-2-[N-[(R)-2-hydroxy-2-phenethyl]-N-[(R)-2-hydroxy-3-phenoxypropyl]amino]propyl]-benzoate hydrochloride, m.p. 70-75° C (ether/methanol), $[\alpha]_D^{25}$ (MEOH) = -48.1° was prepared in an identical manner to the compound described in Example 4 but replacing (S)-phenoxymethyloxirane by its (R) enantiomer. $^1$H nmr ($d_6$-DMSO), free base, ppm

0.84 (3H,d), 2.5-2.87 (5H,m), 3.02 (1H,q), 3.68-3.87 (4H,m), 3.81 (3H,s), 4.56 (1H,t), 4.77 (1H,d, disappears on $D_2O$), 4.86 (1H,d, disappears on $D_2O$), 6.84 (2H,d), 6.86 (1H,dd), 7.19-7.36 (9H,m), 7.79 (2H,d).

$^{13}$C nmr ($d_6$-DMSO), free base, ppm

14.06, 52.01, 53.53, 58.09, 59,86, 68.10, 70.23, 71.40, 114,45, 120.43, 126.23, 126.97, 127.21, 128.02, 129.07, 129.47, 144.12, 146,54, 158.73, 166.34.

EXAMPLE 6

N-Methyl 4-[(R)-2-[N'-[(R)-2-hydroxy-2-phenethyl]-N'-[(S)-2-hydroxy-3-phenoxypropyl]amino]propyl]-benzamide

Methyl 4-[(R)-2-[N-[(R)-2-hydroxy-2-phenethyl]-N-[(S)-2-hydroxy-3-phenoxypropyl]amino]propyl]-benzoate (0.4 g) was dissolved in methanolic methylamine and heated at 100° C in an autoclave for 18h. The solvent was evaporated and the residue was chromatographed on kieselgel. Elution with methanol-chloroform (4:96) gave N-methyl 4-[(R)-2-[N'-[(R)-2-hydroxy-2-phenethyl]-N'-[(S)-2-hydroxy-3-phenoxypropyl] amino]propyl]benzamide, m.p. 52-58° C (ether-hexane), $[\alpha]_D^{25}$ (MeOH) = -59.6°.

$^1$H nmr ($d_6$-DMSO), ppm

0.80 (3H,d), 2.4-2.73 (5H,m), 2.78 (3H,d, collapses to singlet on $D_2O$), 2.87-3.04 (2H,m), 3.78-3.87 (3H,m), 4.56 (1H,t), 4.83 (1H,d, disappears on $D_2O$), 4.95 (1H,d, disappears on $D_2O$), 6.88 (2H,d), 6.90 (1H,dd), 7.18-7.35 (9H, m), 7.73 (2H,d), 8.3 (1H,q, disappears with $D_2O$).

EXAMPLE 7

N-Methyl 4-[(R)-2-[N'-[(R)-2-hydroxy-2-phenethyl]-N'-[(R)-2-hydroxy-3-phenoxypropyl]amino]propyl]-benzamide

N-methyl 4-[(R)-2-N'-[(R)-2-hydroxy-2-phenethyl]-N'-[(R)-2-hydroxy-3-phenoxypropyl]amino]propyl]-benzamide, m.p. 52-58° C (ether-hexane), $[\alpha]_D^{25}$ (MeOH) = -76.4° was prepared in an analogous manner to the compound of Example 6 but using the (RRR) enantiomer.

$^1$H nmr ($d_6$-DMSO), ppm

0.81 (3H,d), 2.45-2.84 (6H,m), 2.76 (3H,d, collapses to singlet on $D_2O$), 2.96 (1H,g), 3.72-3.92 (3H,m), 4.56 (1H,t), 4.80 (1H,d, disappears with $D_2O$), 4.86 (1H,d, disappears with $D_2O$), 6.87 (2H,d), 6.92 (1H,dd), 7.17-7.34 (9H,m), 7.69 (2H,d), 8.26 (1H,q, disappears with $D_2O$).

EXAMPLE 8

4-[2-[N-[2-(3-Chlorophenyl)-2-hydroxyethyl]-N-[2-hydroxy-3-phenoxypropyl]amino]propyl]phenoxyacetamide, hydrochloride

A mixture of (R*,R*)-(±)-methyl 4-[N[2-(3-chlorophenyl)-2-hydroxyethyl]aminopropyl]phenoxyacetate (0.94 g) and phenoxymethyloxirane (0.37 g) was heated under reflux in ethanol (100 ml) for 4 days. The solvent was evaporated and the residue chromatographed on kieselgel. Elution with chloroform gave an oil which was dissolved in methanolic ammonia and allowed to stand at ambient temperature for 18h. The solvent was evaporated and the residue chromatographed on kieselgel. Elution with methanol-chloroform (1:99) and treatment of the product with ethereal hydrogen chloride gave 4-[2-[N-[2-(3-chlorophenyl) -2-hydroxyethyl]-N-[2-hydroxy-3-phenoxypropyl]amino]propyl]3 phenoxyacetamide, hydrochloride, m.p. 177-185° C (ether-methanol).

$^1H$ nmr ($CDCl_3$), free base, ppm

0.98 (3H,d), 2.3-3.2 (8H,m), 3.3-4.1 (4H,m), 4.25 (2H,s), 4.5 (1H,m), 6.4 (2H, broad), 6.7-7.4 (13H,m).

EXAMPLE 9

Methyl 4-[2-[N-[2-hydroxy-2-[4-hydroxy-3-hydroxymethylphenyl]ethyl]-N-[2-hydroxy-3-phenoxypropyl]-amino]propyl] benzoate

A mixture of (R*,R*)-(±)-methyl 4-[N-[-2-hydroxy-2-[4-hydroxy-3-hydroxymethylphenyl]ethyl]-aminopropyl]benzoate (2.0 g) and phenoxymethyloxirane (0.84 g) was heated under reflux in ethanol (100 ml) for 45h. The solvent was evaporated and the residue chromatographed on kieselgel. Elution with methanol-chloroform (4:96) gave after trituration with ether-petrol methyl 4-[2-[N-[2-hydroxy-2-[4-hydroxy-3-hydroxymethylphenyl]ethyl]-N-[2-hydroxy-3-phenoxypropyl]amino]propyl]benzoate, m.p. 38-50° C.

$^1H$ nmr ($d_6$-DMSO), ppm

0.83+0.85 (3H, two doublets), 2.5-3.1 (8H,m), 3.7=3.9 (2H,m, + 3H, two singlets), 4.46 (2H,d, collapses to singlet on $D_2O$, + 1H, m), [4.69 (1H,d), 4.82 (1H,d) , 4.89 (1H,t), all disappear with $D_2O$], 6.69 (2H,d), 6.8-7.0 (4H, m), 7.2-7.4 (5H,m), 7.75-7.86 (2H,dd), 9.13 (1H, s, disappears with $D_2O$).

EXAMPLE 10

Methyl 4-[(R)-2-(N-[2-[4-amino-3,5-dichlorophenyl]-2-hydroxyethyl]-N-[(S)-2-hydroxy-3-phenoxypropyl]-amino] propyl]benzoate

(4-Amino-3,5-dichloro)phenacyl bromide (0.41 g) in acetonitrile (20 ml) was added dropwise to a stirred solution of methyl 4-[(R)-2-[(S)-2-hydroxy-3-phenoxypropyl]amino propyl]benzoate (0.5 g) and triethylamine (0.5 g) in acetonitrile (80 ml) under reflux. The mixture was stirred and heated under reflux for 20h. The solvent was evaporated, chloroform added and triethylamine hydrobromide filtered off. The residue was chromatographed on kieselgel. Elution with chloroform gave the aminoacetophenone which was reduced with sodium borohydride in methanol at ambient temperature. The solvent was evaporated, the residue partitioned between ethyl acetate and water and the organic layer dried and evaporated. The residue was chromatographed on kieselgel. Elution with chloroform gave methyl 4-[(R)-2-[N-[2-[4-amino-3,5-dichlorophenyl]-2-hydroxyethyl] -N-[(S)-2-hydroxy-3-phenoxypropyl]amino]propyl]benzoate, m.p. 40-49° C (ether-hexane).

$^1H$ nmr ($d_6$-DMSO), ppm

0.79 + 0.84 (3H, 2 doublets), 2.4-2.85 (5H,m), 2.86-2.93 (1H,m), 3.70-4.0 (4H, m + 3H, s), 4.41 (1H,m), 4.68-4.79 (1H, 2 doublets, disappears with $D_2O$), 4.96 + 5.1 (1H, 2 doublets, disappears with $D_2O$), 5.3 (2H, s, disappears with $D_2O$), 6.83-6.93 (3H,m), 7.13-7.34 (6H,m), 7.80-7.84 (2H,m).

EXAMPLE 11

Methyl 4-[2-[N-[2-(3-chlorophenyl)-2-hydroxyethyl]-N-[2-hydroxy-3-(4-carboxamidomethyl)phenoxypropyl]-

amino]propyl] phenoxyacetate, hydrochloride

A mixture of ($R^*$,$R^*$)-(±)-Methyl 4-[N-[2-hydroxy-(3-chloro) phenethyl]aminopropyl]phenoxyacetate (from 4.59 g of hydrochloride salt) and 4-(carboxamidomethyl)phenoxymethyloxirane (2.07 g) was heated under reflux in methanol containing triethylamine (5 ml) for 36h. The solvent was evaporated and the residue chromatographed on kieselgel. Elution with methanol-dichloromethane (15:85) gave an oil which was converted to methyl 4-[2-[N-[2-(3-chlorophenyl)-2-hydroxyethyl]-N-[2-hydroxy-3-(4-carboxamidomethyl) phenoxypropyl]amino]propyl]phenoxyacetate, hydrochloride, m.p. 82-93 °C.
$^1$H nmr ($d_6$-DMSO), ppm
1.2 (3H, broad), 2.3-3.6 ( 8H, broad), 3.7 (3H,s), 4.0 (3H, broad), 4.58 (1H, broad), 4.8 (2H,s), 5.3 (1H, broad), 6.1-6.8 (2H + 2H, broad, disappears with $D_2O$), 6.9-7.1 (4H,m), 7.2-7.7 (8H,m), 8.7-9.5 (1H, broad, disappears with $D_2O$).

EXAMPLE 12

4-[(R)-3-[N-(R)-2-Hydroxy-2-phenethyl-N-[(S)-2-hydroxy-3-phenoxypropyl]amino]butyl]benzamide

A mixture of 4-[(R)-3-[N-(R)-2-hydroxy-2-phenethyl]amino] butyl]benzamide (0.2 g) and (S)-phenoxymethyloxirane was heated under reflux in ethanol for 4 days. The solvent was evaporated and the residue chromatographed on kieselgel. Elution with methanol-chloroform (2:98) gave 4-[(R)-3-[N-(R)-2-hydroxy-2-phenethyl-N-[(S)-2-hydroxy-3-phenoxypropyl] amino]butyl]benzamide, m.p. 95.5-97 °C (ethyl acetate diisopropyl ether), $[\alpha]_D^{25}$ (MeOH) -46.1 °.
$^1$H nmr ($d_6$-DMSO), ppm
0.91 (3H,d), 1.4-1.6 (1H,m), 1.65-1.85 (1H,m), 2.5-2.69 (5H,m), 2.7-2.91 (2H,m), 3.8-4.0 (3H,m), 4.58 (1H,t), 4.98 (1H,s, disappears with $D_2O$), 5.15 (1H,s, disappears with $D_2O$), 6.85-6.95 (3H,m), 7.1-7.4 (9H,m), 7.78 (2H,d), 7.86 (2H,s, disappears with $D_2O$).

EXAMPLE 13

4-[(R)-2-[Bis-[(S)-2-hydroxy-3-phenoxypropyl]amino]propyl] phenoxyacetamide, hydrochloride

A mixture Of 4-[(R)-2-aminopropyl]phenoxyacetamide (0.19 g) and (S)-phenoxymethyl oxirane (0.28 g) was heated under reflux in ethanol for 48 hours. The solvent was evaporated and the residue chromatographed on silica. Elution with methanol-chloroform (3:97) gave an oil which was treated with ethereal hydrogen chloride to give 4-[(R)-2-[Bis-[(S)-2-hydroxy-3-phenoxypropyl]amino]propyl]phenoxyacetamide, hydrochloride, m.p. 79-82 °, $[\alpha]_D^{25}$ (MeOH) -56.4 °.
$^1$H nmr ($d_6$-DMSO), ppm
1.15-1.26 (3H,m); 2.72-2.87 (1H,m); 3.20-3.38 (2H,m); 3.42-3.65 (3H,m); 3.82-4.10 (5H,m); 4.32-4.53 (4H,m); 6.00-6.18 (2H, m, replaceable by $D_2O$); 6.85-7.06 (8H,m); 7.20-7.50 (8H,m, replaceable by $D_2O$); 8.78 (1H,s, replaceable by $D_2O$).

EXAMPLE 14

Ethyl 4-[(R)-2-[N-[(R)-2-hydroxy-2-phenethyl]-N-[(S)-2-hydroxy-3-phenoxypropyl]amino]propyl]-phenoxyacetate, hydrochloride

A mixture of ethyl 4-[(R)-2-[N-[(R)-2-hydroxy-2-phenethyl] amino]propyl]phenoxyacetate (0.43 g) and (S)-phenoxymethyl oxirane (0.18 g) was heated under reflux in ethanol for 72 hours. The solvent was evaporated and the residue chromatographed on silica. Elution with chloroform gave an oil which was treated with ethereal hydrogen chloride to give ethyl 4-[(R)-2-[N-[(R)-2-hydroxy-2-phenethyl]-N-[(S)-2-hydroxy-3-phenoxypropyl]amino]propyl]phenoxyacetate, hydrochloride, m.p. 48-52 °, $[\alpha]_D^{25}$ (MeOH) -78.7 °.
$^1$H nmr ($d_6$-DMSO), ppm
1.12-1.27 (6H,m); 2.80-2.93 (1H,m); 3.15-3.65 (5H,m); 3.85-4.10 (3H,m); 4.17 (2H,q); 4.45 (1H,m); 4.75 (2H,s); 5.18 (1H,m); 6.14 (1H, m, replaceable by $D_2O$); 6.40 (1H, m, replaceable by $D_2O$); 6.85-7.03 (5H,m); 7.22-7.55 (9H,m); 8.58 (1H, broad, replaceable by $D_2O$).

EXAMPLE 15

4-[(R)-2-[N-[(R)-2-Hydroxy-2-phenethyl]-N-[(S)-2-hydroxy-3-phenoxypropyl]amino]propyl]phenoxyacetamide, hydrochloride, hemihydrate

A mixture of 4-[(R)-2-[N-[(R)-2-hydroxy-2-phenethyl]amino] propyl]phenoxyacetamide (1.1 g) and (S)-phenoxymethyl oxirane (0.55 g) was heated under reflux for 72 hours in ethanol. The solvent was evaporated and the residual oil was chromatographed on silica. Elution with methanol-chloroform (2:98) gave a gum which was treated with ethereal hydrogen chloride to give 4-[(R)-2-[N-[(R)-2-hydroxy-2-phenethyl]-N-[(S)-2-hydroxy-3-phenoxypropyl]amino]propyl] phenoxyacetamide, hydrochloride, hemihydrate, m.p. 85-89°, (ethyl acetate-ether).

$^1$H nmr ($d_6$-DMSO), ppm

1.13-1.30 (3H,m); 2.77-2.92 (1H,m); 3.12-3.68 (5H,m); 3.82-4.10 (3H,m); 5.11-5.28 (1H,m); 6.10-6.20 (1H,m, disappears with $D_2O$); 6.32-6.45 (1H,m, replaceable by $D_2O$); 6.86-7.05 (5H,m); 7.20-7.55 (12H, m, 3H replaceable by $D_2O$); 8.66 (1H, broad, replaceable by $D_2O$).

EXAMPLE 16

Ethyl 4-[(R)-2-[bis-[(S)-2-hydroxy-3-phenoxypropyl]amino] propyl]phenoxyacetate, hydrochloride, hemihydrate

Ethyl 4-[(R)-2-[bis[(S)-2-hydroxy-3-phenoxypropyl]amino] propyl]phenoxyacetate, hydrochloride, hemihydrate was prepared by an analogous procedure to that described in Example 13 but replacing 4-[(R)-2-aminopropyl]phenoxyacetamide by methyl 4-(R)-2-aminopropyl]phenoxyacetate.

$^1$H nmr ($d_6$ = DMSO), ppm

1.15-1.26 (6H,m); 2.72-2.86 (1H,m); 3.09-3.20 (1H,m); 3.25-3.40 (1H,m); 3.45-3.66 (3H,m); 3.82-4.08 (5H,m); 4.13 (2H,q); 4.44 (2H,m); 4.74 (2H,s); 6.08 (2H,m, replaceable by $D_2O$); 6.83-7.00 (9H,m, 1H replaceable by $D_2O$); 7.22-7.37 (6H,m); 8.80 (1H, broad; replaceable by $D_2O$).

EXAMPLE 17

Ethyl 4-[(R)-2-[N-[(R)-2-(3-chlorophenyl)-2-hydroxyethyl]-N-[(S)-2-hydroxy-3-phenoxypropyl]amino]propyl]-phenoxyacetate, hemifumarate

Ethyl 4-[(R)-2-[N-[(R)-2-(3-chlorophenyl)-2-hydroxyethyl]-N-[(S)-2-hydroxy-3-phenoxypropyl]amino]-propyl]phenoxyacetate, hemifumarate, m.p. 48-52°, was prepared by an analogous procedure to that described in Example 14 but replacing ethyl 4-[(R)-2-[N-[(R)-2-hydroxy-2-phenethyl]amino]propyl] phenoxyacetate by ethyl 4-[(R)-2-[N-[(R)-2-(3-chlorophenyl)-2-hydroxyethyl]amino]propyl]phenoxyacetate.

$^1$H nmr ($d_6$-DMSO), ppm

0.77 (3H,d); 1.20 (3H,t); 2.30-2.40 (1H,m); 2.56-3.00 (6H, m); 4.72-4.88 (3H,m); 4.16 (2H,q); 4.61 (1H,t); 4.70 (2H,s); 6.62 (1H,s); 6.80 (2H,s); 6.85-6.94 (3H,m); 7.08 (2H,d); 7.20-7.40 (6H,m).

EXAMPLE 18

4-[(R)-2-[N-[(R)-2-(3-Chlorophenyl)-2-hydroxyethyl]-N-[(S)-2-hydroxy-3-phenoxypropyl]amino]propyl]-phenoxyacetamide, hydrochloride, hemihydrate

4-[(R)-2-[N-[(R)-2-(3-Chlorophenyl)-2-hydroxyethyl]-N-[(S)-2-hydroxy-3-phenoxypropyl]amino]propyl]-phenoxyacetamide, hydrochloride, hemihydrate- $[\alpha]_D^{25}$ (MeoH)-77.4°, was prepared by an analogous procedure to that described in Example 15 but replacing 4-[(R)-2-[N-[(R)-2-hydroxy-2-phenethyl]amino] propyl]-phenoxyacetamide by 4-[(R)-2-[N-[(R)-2-(3-chlorophenyl) -2-hydroxyethyl]amino]propyl]phenoxyacetamide.

$^1$H nmr ($d_6$-DMSO), ppm

1.15-1.27 (3H,m); 2.79-2.92 (1H,m); 3.12-3.65 (5H,m); 3.82-4.10 (3H,m); 4.40 (2H,s); 4.47 (1H,m); 5.21 (1H,m); 6.14 (1H,m, replaceable by $D_2O$); 6.50 (1H,m, replaceable by $D_2O$); 6.90-7.03 (5H,m); 7.22-7.58 (11H, m, 3H replaceable by $D_2O$); 8.45-8.68 (1H, broad, replaceable by $D_2O$).

EXAMPLE 19

4-[(R)-2-[N-[(R)-2-Hydroxy-2-phenethyl]-N-[(S)-2-hydroxy-3-phenoxypropyl]amino]propyl]-phenoxyethylamine, dihydrochloride, hydrate

Borane methyl sulphide complex (0.32 g) in dry tetrahydrofuran (5 ml) was added in a dropwise manner, under a nitrogen atmosphere, to a stirred solution of 4-[(R)-2-[N-[(R)-2-hydroxy-2-phenethyl]-N-[(S)-2-hydroxy-3-phenoxypropyl]-amino]propyl]phenoxyacetamide (1 g) in dry tetrahydrofuran (30 ml). The reaction mixture was stirred and heated under reflux for 17 hours, cooled, treated with methanol (2.5 ml) and heated for a further hour. Hydrogen chloride was bubbled through the cooled solution. The solvent was evaporated and the residual oil was partitioned between 2N sodium hydroxide solution and dichloromethane. The organic layer was washed with water, dried ($MgSO_4$) and evaporated, to an oil which was purified by column chromatography on silica. Elution with methanol-chloroform (6:94) gave a colourless oil which was treated with ethereal hydrogen chloride to give 4-[(R)-2-[N-[(R)-2-hydroxy-2-phenethyl]-N-[(S)-2-hydroxy-3-phenoxypropyl]amino]propyl]phenoxyethylamine, dihydrochloride, hydrate as a crystalline solid, m.p. 99-103° (methanol-ethylacetate).

$^1H$ nmr ($d_6$-DMSO), ppm

1.14-1.20 (3H,m); 2.45-2.60 (4H,m); 2.81 (1H,m); 3.15-3.68 (4H,m); 3.85-4.30 (6H,m, 2H replaceable by $D_2O$); 4.54 (1H, m); 5.29 (1H,m); 6.20 (1H,m, replaceable by $D_2O$); 6.43 (1H, m, replaceable by $D_2O$); 6.90-7.08 (5H,m); 7.25-7.55 (9H,m); 8.40 (1H,s, replaceable by $D_2O$); 9.12 (1H, broad, replaceable by $D_2O$); 13.70 (1H, broad, replaceable by $D_2O$].

EXAMPLE 20

Ethyl 4-[(R)-3-[N-[(R)-2-hydroxy-2-phenethyl]-N-[[S)-2-hydroxy-3-phenoxypropyl]amino]butyl]-phenoxyacetate, hydrochloride

Ethyl 4-[(R)-3-[N-[(R)-2-hydroxy-2-phenethyl]-N-[(S)-2-hydroxy-3-phenoxypropyl]amino]butyl]-phenoxyacetate, hydrochloride was prepared by an analogous procedure to that described in Example 14 but replacing ethyl 4-[(R)-2-[N-[(R)--2-hydroxy-2-phenethyl]amino]propyl]phenoxyacetate by ethyl 4-[(R)-3-[N-[(R)-2-hydroxy-2-phenethyl]amino]butyl]phenoxyacetate.

$^1H$ nmr ($d_6$-DMSO), ppm

1.21 (3H,t); 1.36 (3H,m); 1.88 (1H,m); 2.11 (1H,m); 2.68 (2H,m); 3.21-3.93 (5H,m); 4.03 (2H,m); 4.16 (2H,q); 4.45 (1H,m); 4.73 (2H,s); 5.16 (1H,m); 6.30 (1H,m, replaceable by $D_2O$); 6.53 (1H,m, replaceable by $D_2O$); 6.82-7.06 (5H,m); 7.13-7.52 (9H,m); 8.33 (1H, broad, replaceable by $D_2O$).

EXAMPLE 21

4-[(R)-2-[N-[[R)-2-Hydroxy-2-phenethyl]-N-[(S)-2-hydroxy 3-phenoxypropyl]amino]propyl]phenoxyethanol, hydrochloride, hemihydrate

4-[(R)-2-[N-[(R)-2-Hydroxy-2-phenethyl]-N-[(S)-2-hydroxy-3-phenoxypropyl]amino]propyl]-phenoxyethanol, hydrochloride, hemihydrate, m.p. 68-73°, was prepared by an analogous procedure to that described in Example 14 but replacing ethyl 4-[(R)-2-[N-[(R)-2-hydroxy-2-phenethyl]amino]propyl] phenoxyacetate by 4-[(R)-2-[N-[(R)-2-hydroxy-2-phenethyl] amino]propyl]phenoxyethanol.

$^1H$ nmr ($d_6$-DMSO), ppm

1.20 (3H,m); 2.83 (1H,m); 3.12-3.63 (5H,m); 4.00 (2H,m); 3.81-4.09 (5H,m); 4.46 (1H,m); 4.80 (1H, broad, replaceable by $D_2O$); 5.18 (1H,m); 6.14 (1H, m, replaceable by $D_2O$); 6.37 (1H, m, replaceable by $D_2O$); 6.85-7.01 (5H, m); 7.20-7.52 (9H,m); 8.56 (1H, broad, replaceable by $D_2O$).

EXAMPLE 22

Ethyl 4-[(R)-2-[N-[(R)-2-hydroxy-2-phenethyl]-N-[(R)-2-hydroxy-3-phenoxypropyl]amino]propyl]-phenoxyacetate, hydrochloride

Ethyl 4-[(R)-2-[N-[(R)-2-hydroxy-2-phenethyl]-N-[(R)-2-hydroxy-3-phenoxypropyl]amino]propyl]-phenoxyacetate, hydrochloride, m.p. 49-52°, was prepared by an analogous procedure to that described in Example 14 but replacing (S)-phenoxymethyloxirane by (R)-phenoxymethyloxirane.

$^1H$ nmr ($d_6$-DMSO), ppm

1.12-1.27 (6H,m); 2.78 (1H,m); 3.22-3.70 (5H,m); 4.01 (3H, m); 4.17 (2H,q); 4.52 (1H,m); 4.75 (2H,s); 5.22 (1H,m); 5.96 (1H, m, replaceable by $D_2O$); 6.39 (1H, m, replaceable by $D_2O$); 6.85-7.02 (5H,m); 7.20-7.55 (9H,m); 9.30 (1H, broad, replaceable by $D_2O$).

EXAMPLE 23

Methyl 4-[(R)-2-[N-[(R)-2-hydroxy-2-phenethyl]-N-[(S)-2-hydroxy-3-phenoxypropyl]amino]propyl]-phenoxyacetate, hydrochloride, hemihydrate

A mixture of methyl 4-[(R)-2-[N-[(R)-2-hydroxy-2-phenethyl] amino]propyl]phenoxyacetate (0.85 g) and (S)-phenoxymethyl oxirane (0.37 g) was heated under reflux in methanol (50 ml) for 52 hours. The solvent was evaporated and the residue chromatographed on silica. Elution with chloroform gave an oil which was treated with ethereal hydrogen chloride to give methyl 4-[(R)-2-[N-[(R)-2-hydroxy-2-phenethyl]-N-[(S)-2-hydroxy-3-phenoxypropyl]amino]propyl]phenoxyacetate hydrochloride hemihydrate, m.p. 58-62°.

$^1H$ nmr ($d_6$-DMSO), ppm

1.25 (3H,m); 2.85 (1H,m); 3.20-3.68 (5H,m); 3.70 (3H,s); 3.80-4.13 (3H,m); 4.49 (1H,m); 4.75 (2H,s); 5.23 (1H,m); 6.16 (1H,m, replaceable by $D_2O$); 6.39 (1H,m, replaceable by $D_2O$); 6.88-7.02 (5H,m); 7.25-7.55 (10H,m, 1H replaceable by $D_2O$); 8.80 (1H, broad, replaceable by $D_2O$).

EXAMPLE 24

4-[(R)-2-[N-[(R)-2-Hydroxy-2-phenethyl]-N-[(S)-2-hydroxy-3-phenoxypropyl]amino]propoxy]-phenoxyacetamide, hydrochloride

4-[(R)-2-[N-[(R)-2-Hydroxy-2-phenethyl]-N-[(S)-2-hydroxy-3-phenoxypropyl]amino]propoxy]-phenoxyacetamide, hydrochloride, m.p. 81-86° (ethyl acetate-ether), was prepared by an analogous procedure to that described in Example 14 but replacing ethyl 4-[(R)-2-[N-[(R)-2-hydroxy-2-phenethyl] amino]propyl]phenoxyacetate by 4-[(R)-2-[N-[(R)-2-hydroxy-2-phenethyl]amino]propoxy]phenoxyacetamide.

$^1H$ nmr ($d_6$-DMSO), ppm

1.75 (3H,m); 3.25-3.75 (3H,m); 3.90-4.50 (9H,m); 5.14 (1H, m); 6.20-6.65 (2H, m, replaceable by $D_2O$); 6.80-7.15 (7H, m); 7.20-7.60 (9H,m, 2H replaceable by $D_2O$); 8.20 (1H, broad, replaceable by $D_2O$).

EXAMPLE 25

Ethyl 4-[(R)-2-[N-[2-(2-benzofuranyl)-2-hydroxyethyl]-N-[(S)-2-hydroxy-3-phenoxypropyl]amino]propyl]-phenoxyacetate, hydrochloride

Ethyl 4-[(R)-2-[N-[2-(2-benzofuranyl)-2-hydroxyethyl]-N-[(S)-2-hydroxy-3-phenoxypropyl]amino]propyl]-phenoxyacetate, hydrochloride, m.p. 56-60°, was prepared by an analogous procedure to that described in Example 14 but replacing ethyl 4-[(R)-2-[N-[(R)-2-hydroxy-2-phenethyl]amino]propyl] phenoxyacetate by ethyl 4-[(R)-2-[N-[2-(2-benzofuranyl)-2-hydroxyethyl]amino]propyl]phenoxyacetate.

$^1H$ nmr ($d_6$-DMSO), ppm

1.11-1.27 (6H,m); 2.70-2.88 (1H,m); 3.25-3.81 (5H,m): 3.91-4.09 (3H,m); 4.16 (2H,q); 4.52 (1H,m); 4.75 (2H,s); 5.48 (1H,m); 6.12 (1H, broad, replaceable by $D_2O$); 6.70 (1H, broad, replaceable by $D_2O$); 6.82-7.03 (6H,m); 7.18-7.38 (6H, m); 7.52-7.70 (2H,m); 9.50 (1H, broad, replaceable by $D_2O$).

EXAMPLE 26

Ethyl 4-[(R)-2-[N-[(R)-2-hydroxy-2-phenethyl]-N-[2-(2-benzofuranyl)-2-hydroxyethyl]amino]propyl]-phenoxyacetate hydrochloride

Ethyl 4-[(R)-2-[N-[(R)-2-hydroxy-2-phenethyl]-N-[2-(2-benzofuranyl)-2-hydroxyethyl]amino]propyl]-phenoxyacetate hydrochloride, m.p. 65-70°, was prepared by an analogous procedure to that described in Example 14 but replacing (S)-phenoxymethyloxirane by 2-benzofuranyloxirane.

$^1H$ nmr ($d_6$-DMSO), ppm

1.15-1.28 (6H,m); 2.75-2.92 (1H,m); 3.30-3.55 (3H,m); 3.75 (2H,m); 4.03 (1H,m); 4.16 (2H,q); 4.75 (2H,s); 5.30 (1H,m); 5.53 (1H,m); 6.40 (1H, m, replaceable by $D_2O$); 6.70 (1H, broad, replaceable by $D_2O$); 6.85-7.00 (3H,m); 7.20-7.70 (11H,m); 9.44 (1H, broad, replaceable by $D_2O$).

EXAMPLE 27

4-[(R)-2-[N-[(R)-2-Hydroxy-2-(4-hydroxyphenyl)-ethyl]-N-[(R)-2-hydroxy-3-phenoxypropyl]amino]propyl]-

phenoxyacetamide

A solution of 4-[(R)-2-[N-[(R)-2-(4-benzyloxyphenyl)-2-hydroxyethyl]-N-[(R)-2-hydroxy-3-phenoxypropyl]-amino]propyl]phenoxyacetamide (0.1 g) in ethyl acetate (60 ml) was hydrogenated at ambient temperature and pressure over 10% palladium on carbon until the theoretical amount of hydrogen was absorbed. The mixture was filtered through celite, evaporated and the residual oil chromatographed on silica. Elution with methanol/chloroform (2:98) gave 4-[(R)-2-[N-[(R)-2-hydroxy-2-(4-hydroxyphenyl)ethyl]-N-[(R)-2-hydroxy-3-phenoxypropyl]amino]propyl]phenoxyacetamide as a colourless oil.
$^1$H nmr ($CDCl_3$), ppm
1.04 (3H,d); 2.50-2.95 (6H,m); 3.15 (1H,m); 3.70-4.12 (3H, m); 4.32-4.60 (6H, m, 3H replaceable by $D_2O$); 5.95 (1H, broad, replaceable by $D_2O$); 6.48 (1H, broad, replaceable by $D_2O$); 6.75-7.29 (13H,m).

EXAMPLE 28

Methyl 4-[2-[N-[2-(4-amino-3,5-dichlorophenyl)-2-hydroxyethyl]-N-[2-hydroxy-3-phenoxypropyl]amino]-propyl]phenoxyacetate, hemifumarate

A mixture of methyl 4-[2-[N-[2-(4-amino-3,5-dichlorophenyl)-2-hydroxyethyl]amino]propyl]-phenoxyacetate (1.0 g) and phenoxymethyloxirane (0.35 g) gas heated under reflux in methanol (80 ml) for 40 hours. The solvent was evaporated and the residue chromatographed on silica. Elution with chloroform gave a colourless oil which was treated with a methanolic solution of fumaric acid to give methyl 4-[2-[N-[2-(4-amino-3,5-dichlorophenyl)-2-hydroxyethyl]-N-[2-hydroxy -3-phenoxypropyl]amino]propyl]phenoxyacetate, hemifumarate as a white solid, m.p. 65-72° (ether).
$^1$H nmr ($d_6$-DMSO), ppm
0.76 (3H,m); 2.25-2.95 (7H,m); 3.15-4.00 (8H,m, 2H replaceable by $D_2O$); 4.44 (1H,m); 4.70 (2H,m); 5.29 (2H,s, replaceable by $D_2O$); 6.62 (1H,s); 6.75-7.31 (11H,m).

EXAMPLE 29

4-[2-[N-[2-(4-Amino-3,5-dichlorophenyl)-2-hydroxyethyl]-N-[2-hydroxy-3-phenoxypropyl]amino]propyl]-phenoxyacetamide

A mixture of 4-[2-[N-[2-(4-amino-3,5-dichlorophenyl)-2-hydroxyethyl]amino]propyl]phenoxyacetamide (0.54 g) and phenoxymethyloxirane (0.2 g) in ethanol (50 ml) was heated under reflux for 48 hours. The solvent was evaporated and the residue chromatographed on silica. Elution with methanol/chloroform (2:98) gave 4-[2-[N-[2-(4-amino-3,5-dichlorophenyl)-2-hydroxyethyl]-N-[2-hydroxy-3-phenoxypropyl]amino]propyl]-phenoxyacetamide as a white solid, m.p. 50-60°.
$^1$H nmr ($d_6$-DMSO), ppm
0.74-0.87 (3H,m); 2.33 (1H,m); 2.50-2.95 (6H,m); 3.65-3.97 (3H,m); 4.31-4.44 (3H,m); 4.70-5.15 (2H,m, replaceable by $D_2O$); 5.28 (2H,m, replaceable by $D_2O$); 6.75-7.45 (13H,m, 2H replaceable by $D_2O$).

The following examples X1 - X14 illustrate the preparation of starting materials.

EXAMPLE X1

Ethyl 4-[(R)-2-[N-[(R)-2-hydroxy-2-phenethyl]amino]propyl] phenoxyacetate

A solution of (R)-phenyloxirane (1.77 g) in ethanol (10 ml) was added dropwise, to a stirred, refluxing solution of methyl 4-[(R)-2-aminopropyl]phenoxyacetate (3.29 g) in ethanol (80 ml). The resulting solution was heated under reflux for 52 hours. The solvent was evaporated and the residual oil was chromatographed on silica gel. Elution with methanol-chloroform (3:97) gave ethyl 4-[(R)-2-[N-[(R)-2-hydroxy-2-phenethyl]amino]propyl]phenoxyacetate as a colourless oil.
$^1$H nmr ($CDCl_3$), ppm
1.00 (3H,d); 1.25 (3H,t); 2.32-3.00 (7H, m, 2H replaceable by $D_2O$); 4.22 (2H,q); 4.35-4.70 (2H,s + 1H, m); 6.65-7.30 (9H,m).

EXAMPLE X2

Ethyl 4-[(R)-2-[N-[(R)-2-(3-chlorophenyl)-2-hydroxyethyl] amino]propyl]phenoxyacetate

Ethyl 4-[(R)-2-[N-[(R)-2-(3-chlorophenyl)-2-hydroxyethyl] amino]propyl]phenoxyacetate was prepared by an analogous procedure to that described in Example X1 but replacing (R)-phenyloxirane by (R)-3-chlorophenyloxirane.
$^1H$ nmr ($CDCl_3$), ppm
1.00 (3H,d); 1.22 (3H,t); 2.35-3.20 (7H,m, 2H replaceable by $D_2O$) 4.25 (2H,q); 4.40-4.70 (2H,s + 1H,m); 6.65-7.40 (8H,m).

EXAMPLE X3

4-[[R)-2-[N-[(R)-2-Hydroxy-2-phenethyl]amino]propyl]phenoxyacetamide

Ammonia gas was bubbled into a solution of ethyl 4-[(R)-2-[N-[(R)-2-hydroxy-2-phenethyl]amino]propyl]-phenoxyacetate (1.45 g) in methanol (50 ml). The resulting solution was allowed to stand for 18 hours and evaporated to give 4-[(R)-2-[N-[(R)-2-hydroxy-2-phenethyl]amino]propyl]phenoxyacetamide as a white solid.
$^1H$ nmr ($CDCl_3$), ppm
1.05 (3H,d); 2.30-3.00 (7H, m, 2H replaceable by $D_2O$); 4.30 (2H,s); 4.55 (1H,t); 6.60-7.40 (11H, m, 2H replaceable by $D_2O$).

EXAMPLE X4

4-[(R)-2-[N-[(R)-2-(3-Chlorophenyl)-2-hydroxyethyl]amino] propyl]phenoxyacetamide

4-[(R)-2-[N-[(R)-2-(3-Chlorophenyl)-2-hydroxyethyl]amino] propyl]phenoxyacetamide was prepared by an analogous procedure to that described in Example X1 but replacing ethyl 4-[(R)-2-[N-[(R)-2-hydroxy-2-phenethyl]amino]propyl]phenoxyacetate by ethyl 4-[(R)-2-[N-[(R)-2-(3-chlorophenyl)-2-hydroxyethyl]amino]-propyl]phenoxyacetate.
$^1H$ nmr ($CDCl_3$), ppm
1.05 (3H,d); 2.30-3.00 (7H, m, 2H replaceable by $D_2O$); 4.40-4.90 (2H,s + 1H,m); 6.80-7.50 (10H, m, 2H replaceable by $D_2O$).

EXAMPLE X5

Ethyl 4-[(R)-2-[N-[2-(2-benzofuranyl)-2-hydroxyethyl]amino] propyl]phenoxyacetate

The title compound was prepared by an analogous procedure to that described in Example X1 but replacing (R)-phenyloxirane by 2-benzofuranyl oxirane.
$^1H$ nmr ($CDCl_3$), ppm
1.00 (3H,d); 1.30 (3H,t); 2.35-3.50 (7H, m, 2H replaceable by $D_2O$); 4.20 (2H,q); 4.40 (2H,s); 4.75 (1H,m); 6.40-7.50 (9H,m).

EXAMPLE X6

Ethyl 4-[(R)-3-[N-[(R)-2-hydroxy-2-phenethyl]amino]butyl] phenoxyacetate

Ethyl 4-[(R)-3-[N-[(R)-2-hydroxy-2-phenethyl]amino]butyl] phenoxyacetate was prepared by an analogous procedure to that described in Example X1 but replacing methyl 4-[(R)-2-aminopropyl]phenoxyacetate by methyl 4-[(R)-3-aminobutyl]phenoxyacetate.
$^1H$ nmr ($CDCl_3$), ppm
1.10 (3H,d); 1.30 (3H,t); 1.70 (2H,m); 2.35-3.00 (5H,m); 3.68 (2H, m, replaceable by $D_2O$); 4.25 (2H,q); 4.50-4.85 (2H,s + 1H,m); 6.70-7.40 (9H,m).

EXAMPLE X7

Methyl 4-[(R)-2-[N-[(R)-2-hydroxy-2-phenethyl]amino]propyl] phenoxyacetate

A mixture of (R)-phenyloxirane (0.86 g) and methyl 4-[(R)-2-aminopropyl]phenoxyacetate (1.6 g) in

dimethylsulphoxide (40 ml) was stirred and heated at 95° C for 24 hours. The reaction mixture was poured into water (100 ml) and extracted into chloroform. The organic layer was washed with water, dried ($MgSO_4$) and evaporated to an oil which was chromatographed on silica. Elution with methanol-chloroform (4:96) gave methyl 4-[(R)-2-[N-[(R)-2-hydroxy-2-phenethyl]amino]propyl]phenoxyacetate as an oil.

$^1H$ nmr ($CDCl_3$), ppm

1.00 (3H,d); 2.35-3.00 (7H, m, 2H replaceable by $D_2O$); 3.70 (3H,s); 4.35-4.65 (2H,s + 1H,m); 6.65-7.30 (9H,m).

EXAMPLE X8

4-[(R)-2-[N-[(R)-2-Hydroxy-2-phenethyl]amino]propyl]phenoxyethanol

Dicyclohexylcarbodiimide (1.1 g) in dry dimethyl formamide was added to a stirred solution of 1-hydroxybenzotriazole (0.73 g), (R)-mandelic acid (0.82 g) and methyl 4-[(R)-2-aminopropyl]phenoxyacetate (1.2 g) in dry dimethyl formamide at 0°. The mixture was allowed to stand at ambient temperature for 20 hours. The solvent was evaporated, the residue taken up in ethyl acetate and filtered. The filtrate was washed successively with sodium carbonate solution, 2N hydrochloric acid, sodium carbonate, water (x 2), dried ($MgSO_4$), filtered and evaporated to a gum which was chromatographed on silica. Elution with chloroform gave N-[(4-carbomethoxymethoxyphenyl)-2-(R)-propyl]-(R)-mandelamide.

$^1H$ nmr ($CDCl_3$), ppm

0.98 (3H,d); 2.50 (2H,d); 3.70 (1H,s, replaceable by $D_2O$); 4.20 (1H,m); 4.45 (2H,s); 4.65 (1H,s + 1H,s, replaceable by $D_2O$); 6.40-7.20 (9H,m).

Borane dimethyl sulphide (1.15 g) was added under nitrogen to a stirred solution of the above material (1.36 g) in dry tetrahydrofuran and the mixture was heated under reflux for 22 hours. The solution was cooled, methanol (3 ml) added and the solution stirred and heated under reflux for one hour. Dry hydrogen chloride was bubbled through the cooled solution, the solvent was evaporated and the residue was partitioned between 2N sodium hydroxide and ethyl acetate. The organic layer was separated, dried ($MgSO_4$) and evaporated to give an oil which was chromatographed on silica. Elution with methanol-chloroform (2:98) gave 4-[(R)-2-[N-[(R)-2-hydroxy-2-phenethyl]amino]propyl]phenoxyethanol as a colourless oil.

$^1H$ nmr ($CDCl_3$), ppm

1.05 (3H,d); 2.50-3.00 (8H, m, 3H replaceable by $D_2O$); 3.80-4.15 (4H,m); 4.60 (1H,m); 6.70-7.20 (4H,q); 7.30 (5H, s).

EXAMPLE X9

Methyl 4-[(R)-2-aminopropyl]phenoxyacetate hydrochloride

A mixture of d-(+)-α-methylbenzylamine (5.23 g) and methyl 4-acetonylphenoxyacetate (9.6 g) in dry benzene was heated under reflux with azeotropic removal of water for 5 hours. The solvent was evaporated, the residue taken up in methanol and hydrogenated over Raney Nickel at 60 psi and ambient temperature for 48 hours. The reaction mixture was filtered through Celite and evaporated to an oil which was dissolved in ether and treated with hydrogen chloride until no further solid precipitated. The solid was filtered and recrystallised from ether-benzene to give methyl 4-[(R)-2-[(R)-1-phenethylamino]propyl]phenoxyacetate hydrochloride as a white solid, m.p. 168-173°.

$^1H$ nmr ($CDCl_3$), free base, ppm

0.90 (3H,d); 1.30 (3H,d); 1.58 (1H,s, replaceable by $D_2O$); 2.40-2.90 (3H,m); 3.60-4.00 (3H,s plus 1H,m); 4.50 (2H,s); 6.60-7.00 (4H,q); 7.20 (5H,s).

A methanolic solution of this hydrochloride (3.3 g) was hydrogenated over 10% palladium on carbon at 50 psi and 50° for 8 hours. The mixture was filtered, evaporated and the residue recrystallised from ethylacetate to give methyl 4-[(R)-2-aminopropyl]phenoxyacetate hydrochloride, m.p. 147°, $[\alpha]_D^{25}$ ($H_2O$) -13.6°.

$^1H$ nmr ($d_6$-DMSO) , ppm

1.20 (3H,d); 2.65-3.50 (3H,m); 3.72 (3H,s); 4.78 (2H,s); 6.83-7.30 (4H,q); 8.30 (3H, broad, replaceable by $D_2O$).

EXAMPLE X10

Methyl 4-[(R)-3-aminobutyl]phenoxyacetate hydrochloride

Methyl 4-[(R)-3-aminobutyl]phenoxyacetate hydrochloride, m.p. 112-116°, $[\alpha]_D^{25}$ (MeOH) + 6.2° was prepared by an analogous procedure to that described in Example X9, but replacing methyl 4-acetonyl phenoxyacetate by methyl 4-[3-oxobutyl]phenoxyacetate.
$^1$H nmr ($d_6$-DMSO), ppm
1.22 (3H,d); 1.80 (2H,m); 2.57 (2H,m); 3.00-3.58 (3H,m, 2H replaceable by $D_2O$); 3.70 (3H,s); 4.78 (2H,s); 6.83-7.25 (4H,q); 8.20 (1H, broad, replaceable by $D_2O$).

EXAMPLE X11

4-[(R)-2-Aminopropoxy]phenoxyacetamide hydrochloride

4-[(R)-2-Aminopropoxy]phenoxyacetamide hydrochloride, m.p. 187-190°, $[\alpha]_D^{25}$ (MeOH) -14.7° was prepared by an analogous procedure to that described in Example X9, but replacing methyl 4-acetonyl-phenoxyacetate by 4-acetonyloxyphenoxyacetamide.
$^1$H nmr ($d_6$-DMSO), ppm
1.32 (3H,d); 3.53 (1H,m); 4.08 (2H,m); 4.38 (2H,s); 7.00 (4H,s); 7.50 (2H, broad, replaceable by $D_2O$); 8.33 (3H, broad, replaceable by $D_2O$).

EXAMPLE X12

Ethyl 4-[(R)-2-[N-[(R)-2-(4-benzyloxyphenyl)-2-hydroxyethyl]amino]propyl]phenoxyacetamide

Ethyl 4-[(R)-2-[N-[(R)-2-(4-benzyloxyphenyl)-2-hydroxyethyl]amino]propyl]phenoxyacetamide was prepared by an analogous procedure to that described in Example X7 but replacing (R)-phenyloxirane by (R)-(4-benzyloxy)phenyloxirane.
$^1$H nmr ($CDCl_3$), ppm
1.10 (3H,d); 2.60-2.85 (7H,m); 3.83 (3H,s); 4.60 (2H,s + 1H,m); 5.10 (2H,s); 6`75-7.35 (13H,m).

EXAMPLE X13

4-[(R)-2-[N-(R)-2-(4-Benzyloxyphenyl)-2-hydroxyethyl]amino] propyl]phenoxyacetamide

4-[(R)-2-[N-(R)-2-(4-Benzyloxyphenyl)-2-hydroxyethyl]amino] propyl]phenoxyacetamide was prepared from ethyl 4-[(R)-2-[N-[(R)-2-(4-benzyloxy)phenyl-2-hydroxyethyl]amino]propyl] phenoxyacetate by an analogous procedure to that described in Example X3.
$^1$H nmr ($CDCl_3$), ppm
1.02 (3H,d); 2.55-3.15 (7H,m); 4.55-4.80 (2H,s + 1H,m); 5.15 (2H,s); 6.80-7.50 (15H,m, 2H replaceable by $D_2O$).

EXAMPLE X14

4-[(R)-2-[N-[(R)-2-(4-Benzyloxyphenyl)-2-hydroxyethyl]-N-[(R)-2-hydroxy-3-phenoxypropyl]amino]propyl]-phenoxyacetamide

4-[(R)-2-[N-[(R)-2-(4-Benzyloxyphenyl)-2-hydroxyethyl]-N-[(R)-2-hydroxy-3-phenoxypropyl]amino]-propyl]phenoxyacetamide was prepared from (R)-phenoxymethyloxirane and 4-[(R)-2-[N-[(R)-2-(4-benzyloxyphenyl)-2-hydroxyethyl]amino]propyl]-phenoxyacetamide by the method described in Example 14.
$^1$H nmr ($CDCl_3$), ppm, free base.
1.05 (3H,d); 2.40-3.10 (9H,m, 2H replaceable by $D_2O$); 3.62-4.10 (3H,m); 4.32-4.70 (2H,s plus 1H,m); 5.08 (2H,s); 6.71-7.40 (20H,m, 2H replaceable by $D_2O$).

Demonstration of Effectiveness of Compounds

a) Anti-Obesity Activity

Female BALB/c mice were trained to feed on powdered oxoid for 1 week. They were then housed in

threes and the compound under study was added to the diet for 3 days. Food was removed at 09.00h and the mice were killed at 11.00h. The parametrial fat pads were weighed as pairs. The result is a mean of 9 values.

| Compound of Example No. | Dose mg/kg diet | Percentage reduction in weight of parametrial fat pads compared with controls |
|---|---|---|
| 4 | 102 | 25 |
| 8 | 55 | 39 |
| 9 | 102 | 13 |
| 11 | 61 | 16 |
| 14 | 27 | 32 |
| 15 | 17 | 27 |
| 16 | 44 | 37 |
| 17 | 25 | 21 |
| 19 | 167 | 15 |
| 22 | 82 | 31 |

b) Effect on Energy Expenditure of Mice

The effect of the compounds on the energy expenditure of mice was demonstrated by means of the following procedure:

Female CD1 mice each weighing approximately 24 g were given food and water ad lib before and during the experiment. The compounds were dissolved in water and these solutions were administered orally to each of 12 mice. A further 12 mice were dosed orally with water. The mice were placed in groups of threes in boxes through which air was drawn and the oxygen content of the air leaving the boxes was measured. The energy expenditure of the mice was calculated for 3 and 21 hours after dosing from the volume of air leaving the boxes and its oxygen content, following the principles described by J.B. de V. Weir, J. Physiol. (London) 109, 1-9 (1949). The results are expressed as a percentage of the rate of energy expenditure of the mice dosed with water.

| Compound of Example No. | Dose mg/kg p.o. | Mean Energy Expenditure | |
|---|---|---|---|
| | | (0-3h) | (0-21h) |
| 1 | 24.7 | 132 | 107 |
| 2 | 30.3 | 122 | 97 |
| 3 | 30.4 | 133 | 109 |
| 4 | 25.5 | 145 | 120 |
| 5 | 25.0 | 136 | 112 |
| 6 | 23.1 | 126 | 110 |
| 7 | 23.1 | 119 | 102 |
| 8 | 27.5 | 158 | 122 |
| 9 | 25.5 | 111 | 106 |
| 10 | 27.4 | 121 | 122 |
| 11 | 30.6 | 120 | 112 |
| 12 | 9.3 | 111 | 103 |
| 13 | 27.3 | 140 | 112 |
| 14 | 27.2 | 144 | 120 |
| 15 | 6.6 | 151 | 113 |
| 19 | 27.8 | 146 | 120 |

c) Effect on Energy Expenditure of Rats

The effect of the compounds on the energy expenditure of rats was demonstrated by means of the following procedure:

Male Sprague-Dawley rats each weighing between 170-200 g were deprived of food for 16 hours before, and during the experiment. Water was provided *ad lib* at all times. The compounds were administered orally in water to 3 or 4 rats. A further 4 rats were dosed orally with water. The rats were placed in boxes through which air was drawn and the oxygen content of the air leaving the boxes was measured. The energy expenditure of the rats was calculated for 3 hours and for 21 hours after dozing from the volume of air leaving the boxes and its oxygen content, following the principles described by J.B. de V. Weir, *J. Physiol.* (London) 109, 1-9 (1949). The results are expressed as a percentage of the rate of energy expenditure of the rats dosed with water.

| Compound of Example No. | Dose mg/kg p.o. | Mean Energy Expenditure (0-3h) | (0-21h) |
|---|---|---|---|
| 1 | 4.93 | 115 | 114 |
| 2 | 6.09 | 114 | 100 |
| 3 | 6.09 | 114 | 100 |
| 4 | 2.5 | 110 | 119 |
| 6 | 4.6 | 112 | 108 |
| 11 | 6.1 | 136 | 111 |
| 12 | 4.6 | 139 | 113 |
| 13 | 5.4 | 127 | 117 |
| 14 | 5.4 | 135 | 118 |
| 15 | 5.2 | 141 | 119 |
| 16 | 2.92 | 120 | 105 |
| 17 | 0.6 | 126 | 109 |
| 18 | 0.55 | 120 | 106 |
| 19 | 2.78 | 112 | 110 |
| 20 | 2.79 | 110 | 107 |
| 21 | 1.02 | 120 | 107 |
| 22 | 1.63 | 129 | 111 |
| 23 | 1.08 | 146 | 122 |
| 24 | 10.6 | 126 | 115 |
| 25 | 1.17 | 142 | 117 |
| 26 | 1.11 | 134 | 114 |
| 27 | 24.7 | 131 | 117* |
| 28 | 31.8 | 131 | 122* |
| 29 | 28.1 | 129 | 116* |

* indicates 0-19h only

d) Hypoglycaemic activity

Female CFLP mice, weighing approximately 25 g, were fasted for 24 hours prior to the study. The compounds under study were administered orally as an aqueous solution to each of 6 mice. 30 minutes later a blood sample (20 μl) was obtained from the tail for the analysis of blood glucose. Immediately after taking this blood sample, glucose (1 g/kg body weight) was administered subcutaneously to each mouse. 6 mice were given water as a control. Blood samples were then obtained from each mouse at 30 minute intervals for 120 minutes.

Compounds that produced a significant ($p<0.05$) reduction of blood glucose, compared with control mice given water, at any time interval, were considered active. The area under the blood glucose curve over

the 2 hour period after the administration of the glucose was calculated for each compound and compared with the value for control animals.

| Compounds of Example No. | Dose µmol/kg | % Reduction in Area under Blood Glucose Curve |
|---|---|---|
| 1 | 5 | 28 |
| 3 | 5 | 12 |
| 4 | 2.5 | 12 |
| 5 | 2.5 | 38 |
| 6 | 2.5 | 13 |
| 7 | 12.5 | 18 |
| 8 | 0.5 | 50 |
| 9 | 12.5 | 34 |
| 10 | 2.5 | 31 |
| 11 | 2.5 | 57 |
| 12 | 2.5 | 28 |
| 13 | 5.0 | 20 |
| 14 | 2.5 | 42 |
| 17 | 2.5 | 45 |
| 18 | 0.1 | 43 |
| 19 | 12.5 | 40 |
| 25 | 0.1 | 34 |

e) Effect of Compounds on Isolated Rat Atria

Sprague-Dawley male rats (300-400 g) are killed and the right and left atria dissected out independently and mounted on a combined tissue holder/electrode. The tissues are then immersed in a glass bath containing Krebs solution maintained at 32° C. The rate of the spontaneously beating right atrium (via an isometric tranducer and a ratemeter) and the tension of the electrically paced left atrium (via an isometric transducer) are recorded on an M19 chart recorder.

After an initial stabilization period, the tissues are exposed to a maximal concentration of isoprenaline ($10^{-6}$M). The tissues are then washed several times until rate and tension return to baseline level. A dose-response curve to the test compound is then carried out.

Compounds of Example Nos. 1, 4, 8, 13 and 14 had no effect on rate or tension up to $3x10^{-5}$M.

## Claims

**1.** A compound of the general formula (I):

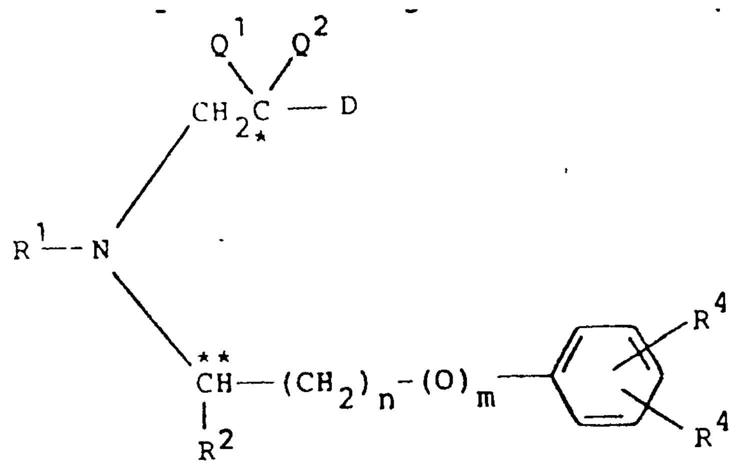

(I)

or a pharmaceutically acceptable salt thereof,
in which

$R^1$

represents a 2-hydroxy-2-(benzofuran-2-yl)ethyl group or a radical of the general formula (a) or (b):

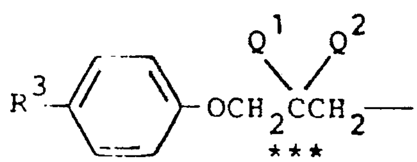

or

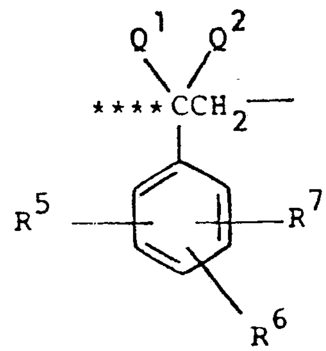

(a) (b)

$R^2$

represents a hydrogen atom or a methyl group,

D

represents either a benzofuran-2-yl moiety or a radical of general formula (c):

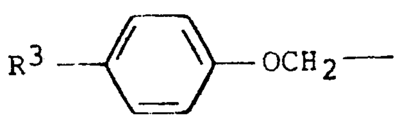

(c)

$R^3$

represents a hydrogen atom or a $CH_2CONH_2$ group, and where there are two $R^3$s in the compound these are the same or different, provided that there is a maximum of one $CH_2CONH_2$ group in the compound,

$R^4$

represents a radical $-(CH_2)_aCOOH$, $-O(CH_2)_bCOOH$, or $-C(R^8)=C(R^9)COOH$ or an ester or an amide, lower alkylamide, di-lower alkylamide or saturated 5- or 6-membered ring amide derivative of one of these groups, or $-SO_2NR^{10}R^{11}$, $-(CH_2)_aCH_2X$, or $-O(CH_2)_bCH_2X$ in which

a

represents zero or an integer from 1 to 6,

b

represents an integer from 1 to 6,

$R^8$ and $R^9$,

which may be the same or different, each represents a hydrogen atom or a methyl group,

$R^{10}$ and $R^{11}$,

which may be the same or different, each represents a hydrogen atom or a lower alkyl radical or, together with the nitrogen atom to which they are attached, form a saturated 5- or 6- membered ring, and

X

represents a hydrogen atom or a lower alkyl radical, a hydroxy group or an ester thereof,a lower alkoxy radical, an unsubstituted or substituted benzyloxy radical or a (lower alkyl)carbonyl radical, or a radical $NR^{10}R^{11}$ in which $R^{10}$ and $R^{11}$ have the meanings given above,

$R^{4\prime}$

represents a hydrogen or a halogen atom or a lower alkyl or lower alkoxy radical, a $CF_3$ group or a hydroxy group or an ester thereof,

$R^5$
represents a hydrogen or halogen atom, a hydroxy or hydroxymethyl group or an ester of either of these groups, or a group $CF_3$, $NH_2$, $NHR^{12}$, $NHCOOR^{12}$, or $CH_2SO_2R^{12}$ in which $R^{12}$ represents a lower alkyl radical,
$R^6$
represents a hydrogen or halogen atom, or a hydroxy group or ester thereof,
$R^7$
represents a hydrogen or halogen atom,
n
represents 1 or 2,
m
represents 0 or 1,
provided that n + m = 1 or 2, and
$Q^1$
represents a hydrogen atom and
$Q^2$
represents a hydroxy group or a pharmaceutically acceptable in-vivo hydrolysable ester thereof or
$Q^1$ and $Q^2$
together represent an oxo group,the two $Q^1Q^2$ pairs in the compound being the same or different, provided there is a maximum of one $Q^1Q^2$ oxo group in the compound;
with the proviso that when $R^1$ represents a radical of the abovementioned formula (a), wherein $R^3$ and $Q^1$ each represent a hydrogen atom and $Q^2$ represents a hydroxy group or a pharmaceutically acceptable in-vivo hydrolysable ester thereof, or $R^1$ represents a radical of the abovementioned formula (b) wherein $R^5$ represents a hydrogen or halogen atom or a trifluoromethyl group and $R^6$, $R^7$ and $Q^1$ each represent a hydrogen atom and $Q^2$ represents a hydroxy group or an in-vivo hydrolysable ester thereof; and $R^2$ represents a hydrogen atom or a methyl group; and
D represents a radical of the abovementioned formula (c) wherein $R^3$ represents a hydrogen atom; $Q^1$ represents a hydrogen atom and $Q^2$ represents a hydroxy group or an in-vivo hydrolysable ester thereof;
$R^4$ represents a para substituent on the phenyl ring, relative to the $-(O)_m-$ moiety, and represents either a radical $-(CH_2)_aCO_2H$, $-C(R^8)=C(R^9)CO_2H$, or an ester or amide, lower alkylamide or di- lower alkylamide of one of these groups; or $-SO_2NR^{10}R^{11}$; $-(CH_2)_aCH_2X$, wherein X represents hydroxy or a (lower alkyl)carbonyl radical;
$-O(CH_2)_bCH_2X$ wherein X represents hydroxy, lower alkoxy or an unsubstituted benzyloxy group;
and a represents zero or an integer from 1 to 6;
and b represents an integer from 1 to 6;
and $R^8$ represents a hydrogen atom or a methyl group;
and $R^9$, $R^{10}$ and $R^{11}$ each represents a hydrogen atom;
and n represents 1 or 2 and m represents zero; then $R^{4\prime}$ does not represent a hydrogen atom.

2. A compound as claimed in claim 1, wherein in each of the radicals (a) and(b) represented by $R^1$, $Q^1$ represents a hydrogen atom and $Q^2$ represents a hydroxy group or a pharmaceutically acceptable in-vivo hydrolysable ester thereof.

3. A compound as claimed in claim 1 or claim 2, of

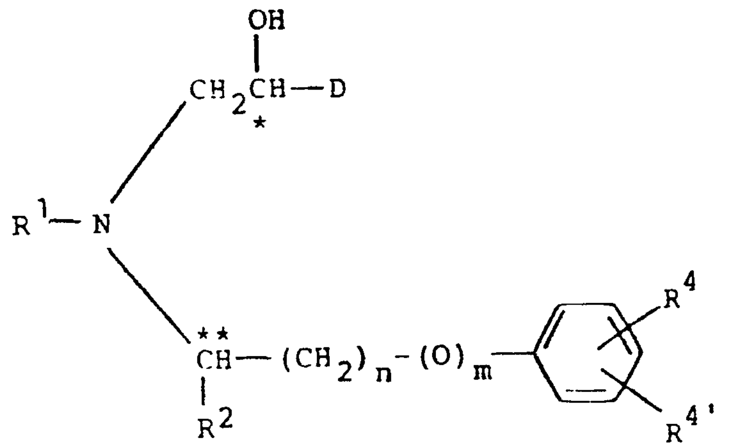

(I')

formula (I'): or a pharmaceutically acceptable salt thereof,
in which $R^1$ represents a 2-hydroxy-2-(benzofuran-2-yl) ethyl group or a radical of the general formula (a') or (b'):

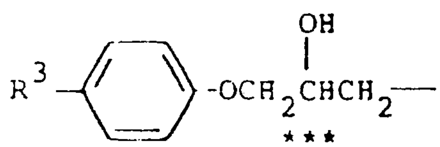 or 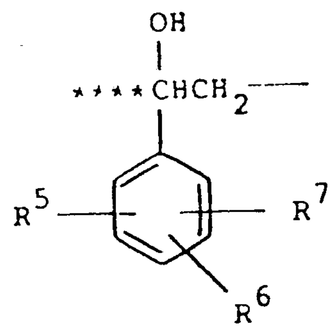

(a') (b')

and $R^2$, $R^3$, $R^4$, $R^{4'}$, $R^5$, $R^6$, $R^7$, m and n are as defined in respect of formula (I) in claim 1; and D represents a benzofuran-2-yl moiety or a radical of formula (c) as defined in claim 1.

**4.** A compound as claimed in any one of claims 1 to 3, wherein $R^1$ represents a 2-hydroxy-2-(benzofuran-2-yl)ethyl group or a moiety of formula (a') or (b'):

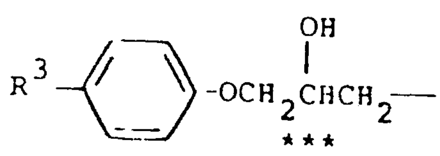 or 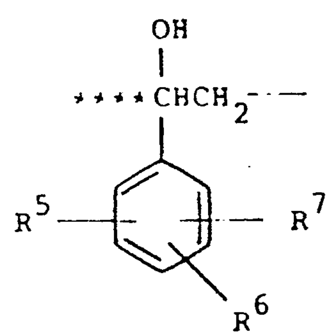

(a') (b')

$R^3$, $R^6$ and $R^7$ are as defined in relation to formula (I) in claim 1, and
$R^5$
represents a hydrogen or halogen atom, a hydroxy or hydroxymethyl group or an ester of either of these groups, or a group $NH_2$ or $NHR^{12}$ in which

$R^{12}$
represents a lower alkyl radical.

5. A compound as claimed in any one of claims 1 to 4, wherein the moiety of formula (b) is one of the following groups:

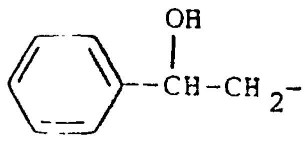 , 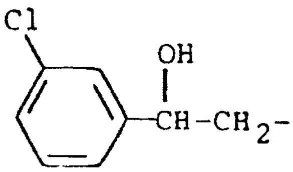

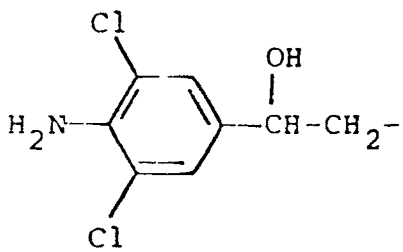 , 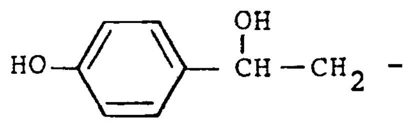

6. A compound as claimed in any one of claims 1 to 5, wherein $R^4$ is in the <u>para</u> position.

7. A compound as claimed in any one of claims 1 to 6, wherein $R^4$ represents a radical of formula $-O-(CH_2)_bCOOH$ or an ester or amide derivative thereof.

8. A compound as claimed in any one of claims 1 to 7, wherein b represents 1.

9. A compound as claimed in claim 1, selected from the group consisting of:

methyl 4-[(R)-2-[bis-[(S)-2-hydroxy-3-phenoxypropyl]amino]propyl]benzoate;

methyl 4-[(R)-2-[bis-[(R)-2-hydroxy-3-phenoxypropyl]amino]propyl]benzoate;

N-methyl 4-[(R)-2-[bis-[(S)-2-hydroxy-3-phenoxypropyl]amino]propyl]benzamide;

methyl 4-[(R)-2-[N-[(R)-2-hydroxy-2-phenethyl]-N-[(S)-2-hydroxy-3-phenoxypropyl]amino]propyl]-benzoate;

methyl 4-[(R)-2-[N-[(R)-2-hydroxy-2-phenethyl]-N-[(R)-2-hydroxy-3-phenoxypropyl]amino]propyl]-benzoate;

N-methyl 4-[(R)-2-[N'-[(R)-2-hydroxy-2-phenethyl]-N'-[(S)-2-hydroxy-3-phenoxypropyl]amino]propyl] benzamide;

N-methyl 4-[(R)-2-[N'-[(R)-2-hydroxy-2-phenethyl]-N'-[(R)-2-hydroxy-3-phenoxypropyl]amino]propyl] benzamide;

4-[2-[N-[2-(3-chlorophenyl)-2-hydroxyethyl]-N-[2-hydroxy-3-phenoxypropyl]amino]propyl]-phenoxyacetamide;

methyl 4-[2-[N-[2-hydroxy-2-[4-hydroxy-3-hydroxymethylphenyl]ethyl]-N-[2-hydroxy-3-phenoxypropyl] amino]propyl]benzoate;

methyl 4-[(R)-2-[N-[2-[4-amino-3,5-dichlorophenyl]-2-hydroxyethyl]-N-[(S)-2-hydroxy-3-phenoxypropyl]-amino]propyl]benzoate;

methyl 4-[2-[N-[2-(3-chlorophenyl)-2-hydroxyethyl]-N-[2-hydroxy-3-(4-carboxamidomethyl)-phenoxypropyl]amino]propyl]phenoxyacetate;

4-[(R)-3-[N-(R)-2-hydroxy-2-phenethyl-N-[(S)-2-hydroxy-3-phenoxypropyl]amino]butyl]benzamide;

4-[(R)-2-[bis-(S)-2-hydroxy-3-phenoxypropyl]amino]propyl]phenoxyacetamide;

ethyl 4-[(R)-2-[N-[(R)-2-hydroxy-2-phenethyl]-N-[(S)-2-hydroxy-3-phenoxypropyl]amino]propyl] phenoxyacetate;

4-[(R)-2-[N-[(R)-2-hydroxy-2-phenethyl]-N-[(S)-2-hydroxy-3-phenoxypropyl]amino]propyl] phenoxyacetamide;

ethyl 4-[(R)-2-bis-[(S)-2-hydroxy-3-phenoxypropyl] amino]propyl]phenoxyacetate;

ethyl 4-[(R)-2-[N-[(R)-2(3-chlorophenyl) -2-hydroxyethyl]-N-[(S)-2-hydroxy-3-phenoxypropyl]amino]-propyl] phenoxyacetate;

4-[(R)-2-[N-[(R)-2-(3-chlorophenyl)-2-hydroxyethyl]-N-[(S)-2-hydroxy-3-phenoxypropyl]amino]propyl] phenoxyacetamide;

4-[(R)-2-[N-[(R)-2-hydroxy-2-phenethyl]-N-[(S)-2-hydroxy-3-phenoxypropyl]amino]propyl]-phenoxyethylamine;

ethyl 4-[(R)-3-[N-[(R)-2-hydroxy-2-phenethyl]-N-[(S)-2-hydroxy-3-phenoxypropyl]amino]butyl]-phenoxyacetate;

4-[(R)-2-[N-[(R)-2-hydroxy-2-phenethyl]-N-[(S)-2-hydroxy-3-phenoxypropyl]amino]propyl]-phenoxyethanol;

ethyl 4-[(R)-2-[N-[(R)-2-hydroxy-2-phenethyl]-N-[(R)-2-hydroxy-3-phenoxypropyl]amino]propyl]-phenoxyacetate;

methyl 4-[(R)-2-[N-[(R)-2-hydroxy-2-phenethyl]-N-[(S) -2-hydroxy-3-phenoxypropyl]amino]propyl]-phenoxyacetate;

4-[(R)-2-[N-[(R)-2-hydroxy-2-phenethyl]-N-[(S)-2-hydroxy-3-phenoxypropyl]amino]propoxy]-phenoxyacetamide;

ethyl 4-[(R)-2[N-[2-(2-benzofuranyl)-2-hydroxyethyl] -N-[(S)-2-hydroxy-3-phenoxypropyl]amino]propyl] phenoxyacetate;

ethyl 4-[(R)-2-[N-[(R)-2-hydroxy-2-phenethyl]-N-[2-(2-benzofuranyl)-2-hydroxyethyl]amino]propyl] phenoxyacetate;

4-[(R)-2-[N-[(R)-2-hydroxy-2-(4-hydroxyphenylethyl]-N-[(R)-2-hydroxy-3-phenoxypropyl]amino]propyl] phenoxyacetamide;

methyl 4-[2-[N-[2-(4-amino-3,5-dichlorophenyl)-2-hydroxyethyl]-N-[2-hydroxy-3-phenoxypropyl]amino] propyl]phenoxyacetate; and

4-[2-[N-[2-(4-amino-3,5-dichlorophenyl)-2-hydroxyethyl] -N-[2-hydroxy-3-phenoxypropyl]amino]propyl] phenoxyacetamide; or a pharmaceutically acceptable salt thereof.

**10.** A process for the preparation of a compound of the general formula (I) as defined in claim 1, or a pharmaceutically acceptable salt thereof, characterized in that the process comprises:
A) reacting a compound of the general formula (II):

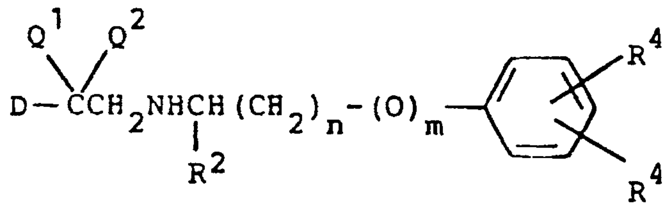

(II)

in which $R^2$, D, $R^4$, $R^{4'}$, n, m, $Q^1$ and $Q^2$ are as defined in relation to formula (I) in claim 1 with (i) a compound of the general formula

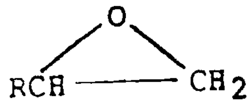

(III)

in which R represents a benzofuran-2-yl moiety or a group of the general formula

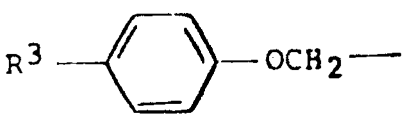

or

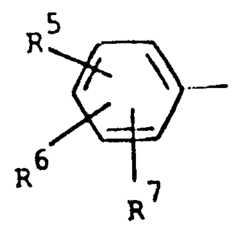

in which $R^3$, $R^5$, $R^6$ and $R^7$ are as defined in relation to formula (I) in claim 1, or (ii) a compound of the general formula (IV):

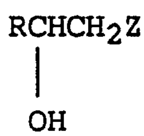

(IV)

in which R is as defined in relation to formula (III) and Z represents a leaving group, or an ester thereof; or

B) for the preparation of a compound of the general formula (I) in which there is one oxo group represented by $Q^1 + Q^2$, or a pharmaceutically acceptable salt thereof, reacting a compound of the general formula (II), as defined above, provided that $Q^1$ and $Q^2$ do not represent an oxo group, with a compound of the general formula (V):

$RCOCH_2Z$ (V)

in which R and Z are as defined in relation to formula (III) and, if desired, converting the resulting compound of the general formula (VI):

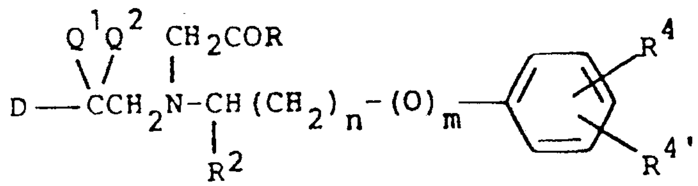

(VI)

in which R, $R^2$, D, $R^4$, $R^{4'}$, n, m, $Q^1$ and $Q^2$ are as defined in relation to formula (I) in claim 1, and provided that $Q^1$ and $Q^2$ do not represent an oxo group, into another compound of the general formula (VI) (or I); or

C) reacting a compound of the general formula (IX):

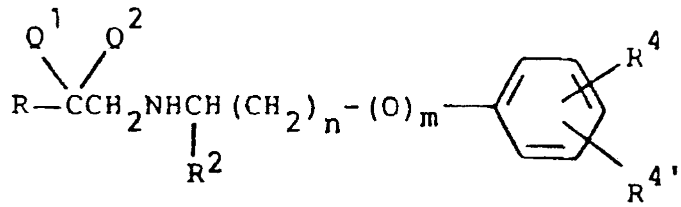

(IX)

in which $R^2$, $R^4$, $R^{4'}$, n, m, $Q^1$ and $Q^2$ are as defined in relation to formula (I) in claim 1 and R is as defined in relation to formula (III), with the hereinbefore defined compound of the general formula (VII)a or (VII)b, providing that if $Q^1$ and $Q^2$ together represent a carbonyl group then compound (IX) is reacted with compound (VII)a; or

D) reacting a compound of the abovementioned general formula (II):

(i) with a compound of the general formula (X):

R-CO.CHO (X)

in which R, is as defined in relation to formula (III) and subsequently treating with a reducing agent, or

(ii) with a compound of the general formula (XI):

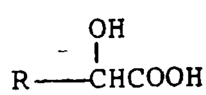

(XI)

in which R is as defined in relation to formula (III) and subsequently reducing the resulting hydroxyamide; or

E) reacting a compound of the general formula (XII):

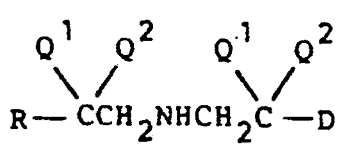

(XII)

in which D, $Q^1$ and $Q^2$ are as defined in relation to formula (I) in claim 1 and R is as defined in

relation to formula (III),

(i) with a compound of the general formula (XIII):

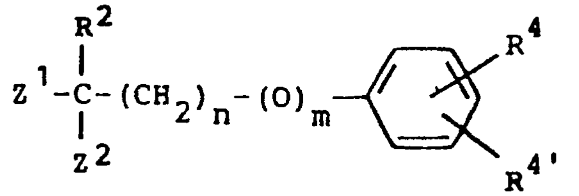

(XIII)

in which $R^2$, $R^4$, $R^{4\prime}$, n and m are as defined in relation to formula (I) in claim 1 and $Z^1$ represents a leaving group and $Z^2$ represents a hydrogen atom, or

(ii) with a compound of the general formula (XIII) in which $R^2$, $R^4$, $R^{4\prime}$, n and m are as defined in relation to formula (I) in claim 1 and $Z^1$ and $Z^2$ together represent an oxo group; and if necessary treating with a reducing agent; or

F) reducing a compound of the abovementioned general formula (VI) in which R, $R^2$, D, $R^4$, $R^{4\prime}$, n, m, $Q^1$ and $Q^2$ are as defined in relation to formula (I) in claim 1, or a salt thereof; and thereafter, if necessary, converting a compound of formula (I) to another compound of formula (I) and/or forming a pharmaceutically acceptable salt of the compound so formed.

**11.** A compound of formula (I) as defined in claim 1, or a pharmaceutically acceptable salt thereof, for use as an active therapeutic substance.

**12.** A compound of formula (I) as defined in claim 1, or a pharmaceutically acceptable salt thereof, for use in the treatment of obesity and/or hyperglycaemia in humans or non-human animals.

**13.** A pharmaceutical composition comprising a compound of formula (I) as defined in claim 1, or a pharmaceutically acceptable salt thereof, with a pharmaceutically acceptable carrier therefore.

**14.** A method for improving the weight gain and/or improving the feed utilisation efficiency and/or increasing lean body mass of livestock, which method comprises the administration to livestock of an effective non-toxic amount of a compound of formula (I) as defined in claim 1, or a veterinarily acceptable salt thereof.

## Revendications

**1.** Composé caractérisé par la formule générale (I) :

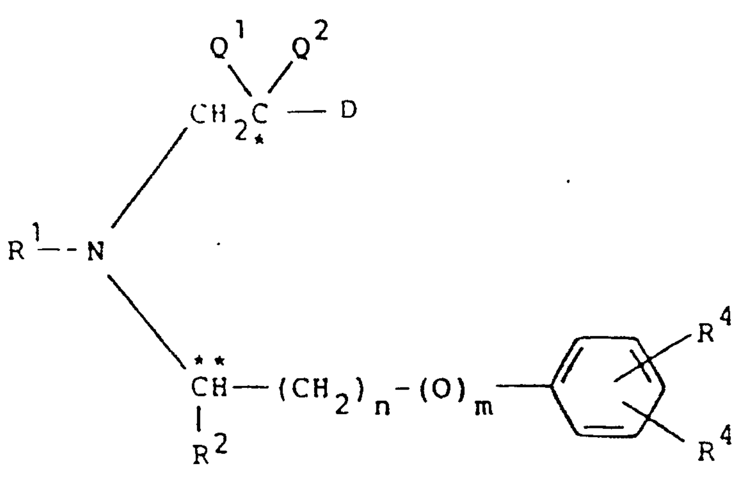

(I)

ou un sel de celui-ci acceptable du point de vue pharmaceutique,
dans laquelle
$R^1$ représente un groupe 2-hydroxy-2-(benzofuran-2-yl)éthyle ou un radical de formule générale (a) ou (b) :

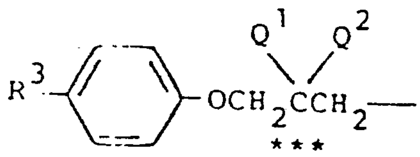

(a)

ou

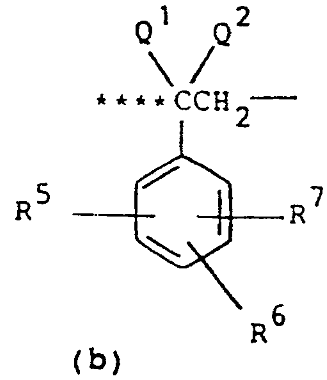

(b)

$R^2$
représente un atome d'hydrogène ou un groupe méthyle,
D
représente une partie benzofuran-2-yle ou un radical de formule générale (c) :

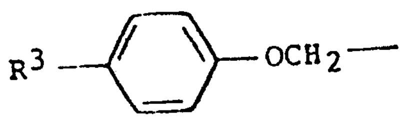

(c)

$R^3$
représente un atome d'hydrogène ou un groupe $CH_2CONH_2$ et lorsqu'il y a deux groupes $R^3$ dans le composé, ceux-ci sont identiques ou différents, à condition qu'il y ait au maximum un groupe $CH_2CONH_2$ dans le composé,
$R^4$
représente un radical $-(CH_2)_aCOOH$, $-O(CH_2)_bCOOH$ ou $-C(R^8)=C(R^9)COOH$ ou un dérivé ester ou amide, alkylamide inférieur, di-alkylamide inférieur ou amide à cycle saturé à 5 ou 6 chaînons de l'un de ces groupes, ou $-SO_2NR^{10}R^{11}$, $-(CH_2)_aCH_2X$ ou $-O(CH_2)_bCH_2X$, dans lesquels
a
représente 0 ou un entier de 1 à 6, b
représente un entier de 1 à 6,
$R^8$ et $R^9$
qui peuvent être identiques òu différents, représentent chacun un atome d'hydrogène ou un groupe méthyle,
$R^{10}$ et $R^{11}$,
qui peuvent être identiques ou différents, représentent chacun un atome d'hydrogène ou un groupe alkyle inférieur, ou bien ils forment ensemble avec l'atome d'azote auxquels ils sont attachés, un cycle saturé à 5 ou 6 chaînons, et
X
représente un atome d'hydrogène ou un radical alkyle inférieur, un groupe hydroxy ou un ester de celui-ci, un groupe alcoxy inférieur, un radical benzyloxy substitué ou non-substitué ou un radical alkyl-(inférieur)carbonyle, ou un radical $R^{10}R^{11}$ dans lequel $R^{10}$ et $R^{11}$ sont tels que définis plus haut,
$R^{4'}$
représente un atome d'hydrogène ou d'halogène ou un groupe alkyle inférieur ou un radical alcoxy inférieur, un groupe $CF_3$ ou un groupe hydroxy ou un ester de celui-ci,
$R^5$

représente un atome d'hydrogène ou d'halogène, un groupe hydroxy ou hydroxyméthyle ou un ester de l'un ou l'autre de ces groupes, ou un groupe $CF_3$, $NH_2$ , $NHR^{12}$, $NHCOOR^{12}$ ou $CH_2SO_2R^{12}$ dans lequel $R^{12}$ représente un radical alkyle inférieur,

$R^6$

représente un atome d'hydrogène ou d'halogène, ou un groupe hydroxy ou un ester de celui-ci,

$R^7$

représente un atome d'hydrogène ou d'halogène,

n

représente un ou 2,

m

représente 0 ou 1,

à condition que n + m = 1 ou 2, et

$Q^1$

représente un atome d'hydrogène, et

$Q^2$

représente un groupe hydroxy ou un groupe ester de celui-ci hydrolysable in vivo acceptable du point de vue pharmaceutique, ou bien

$Q^1$ et $Q^2$

représentent ensemble un groupe oxo, les deux paires $Q^1$ et $Q^2$ dans le composé étant identiques ou différents à condition qu'il y ait un maximum d'un groupe oxo $Q^1Q^2$ dans le composé ;

à la condition que lorsque $R^1$ représente un radical de formule (a) mentionnée plus haut, dans laquelle $R^3$ et $Q^1$ représentent chacun un atome d'hydrogène et $Q^2$ représente un groupe hydroxy ou un ester de celui-ci hydrolysable in vivo acceptable du point de vue pharmaceutique, où $R^1$ représente un radical de formule (b) mentionnée plus haut dans laquelle $R^5$ représente un atome d'hydrogène ou d'halogène ou un groupe trifluorométhyle et $R^6$,

$R^7$ et $Q^1$ représentent chacun un atome d'hydrogène et $Q^2$ représente un groupe hydroxy ou un groupe ester de celui-ci hydrolysable in vivo ; et $R^2$ représente un atome d'hydrogène ou un groupe méthyle ; et D représente un radical de formule (c) mentionnée plus haut dans laquelle $R^3$ représente un atome d'hydrogène ; $Q^1$ représente un atome d'hydrogène et $Q^2$ représente un groupe hydroxy ou un groupe ester de celui-ci hydrolysable in vivo ;

$R^4$ représente un substituant para sur le cycle phényle, par rapport à la partie $-(O)_m-$ , et représente un radical $-(CH_2)_aCO_2H$ , $-C(R^8)=C(R^9)CO_2H$ ou un dérivé ester ou amide, alkylamide inférieur ou di-alkylamide inférieur de l'un de ces groupes ; ou un radical $-SO_2NR^{10}R^{11}$ ;

$-(CH_2)_aCH_2X$ dans lequel X représente un groupe hydroxy ou un radical alkyl(inférieur)carbonyle ;

$-O(CH_2)_bCH_2X$ dans lequel X représente un groupe hydroxy, alcoxy inférieur ou benzyloxy non-substitué ;

et a représente 0 ou un entier de 1 à 6,

et b représente un entier de 1 à 6,

et $R^8$ représente un atome d'hydrogène ou un groupe méthyle,

et $R^9$, $R^{10}$ et $R^{11}$ représentent chacun un atome d'hydrogène ;

et n représente 1 ou 2 ; et m représente 0 ; alors $R^{4'}$ ne représente pas un atome d'hydrogène.

**2.** Composé suivant la revendication 1, caractérisé en ce que dans chacun des radicaux (a) et (b) représenté par $R^1$, $Q^1$ représente un atome d'hydrogène et $Q^2$ représente un groupe hydroxy ou un ester de celui-ci hydrolysable in vivo acceptable du point de vue pharmaceutique.

**3.** Composé suivant la revendication 1 ou la revendication 2, caractérisé par la formule (I') :

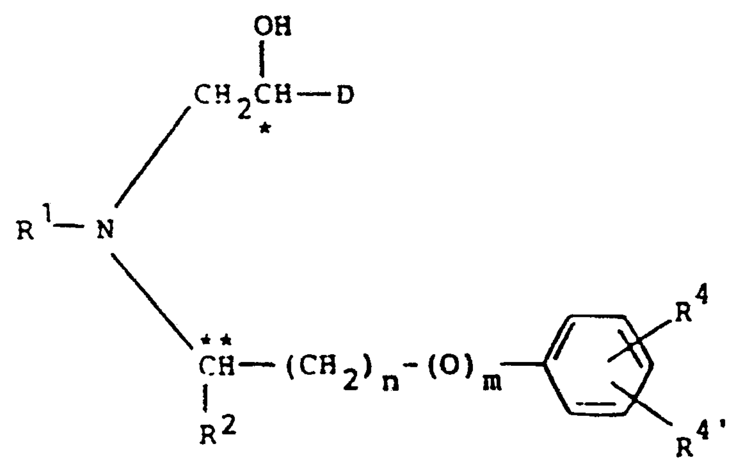

(I')

ou un sel de celui-ci acceptable du point de vue pharmaceutique, dans laquelle $R^1$ représente un groupe 2-hydroxy-2-(benzofuran)2-yl)éthyle ou un radical de formule générale (a') ou (b') :

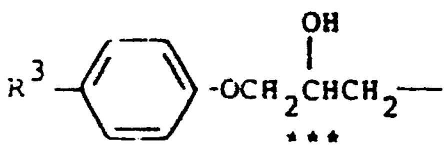

ou

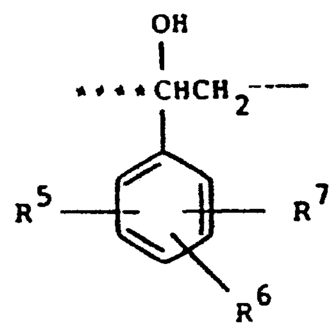

(a') (b')

et $R^2$, $R^3$, $R^4$, $R^{4'}$, $R^5$ , $R^6$, $R^7$, m et n sont tels que définis à propos de la formule (I) dans la revendication 1 ; et D représente une partie benzofuran-2-yle ou un radical de formule (c) telle que définie dans la revendication 1.

4. Composé suivant l'une quelconque des revendications 1 à 3, caractérisé en ce que $R^1$ représente un groupe 2-hydroxy-2-(benzofuran-2-yl)éthyle ou un groupe de formule (a') ou (b') :

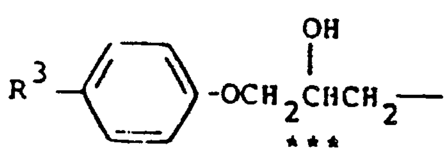

ou

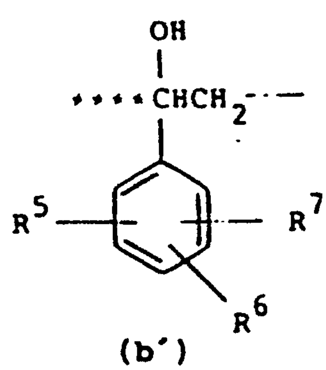

(a') (b')

$R^3$ , $R^°$ et R' sont tels que définis à propos de la formule (I) dans la revendication 1, et
$R^5$
représente un atome d'hydrogène ou d'halogène, un groupe hydroxy ou hydroxyméthyle ou un ester de l'un ou l'autre de ces groupes, ou un groupe $NH_2$ ou $NHR^{12}$ où
$R^{12}$
représente un radical alkyle inférieur.

5. Composé suivant l'une quelconque des revendications 1 à 4, caractérisé en ce que la partie de formule (b) est représentée par l'un des groupes suivants :

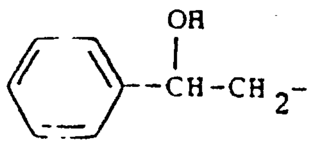
,
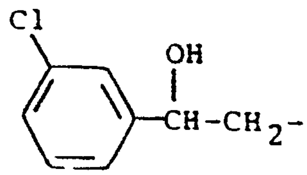

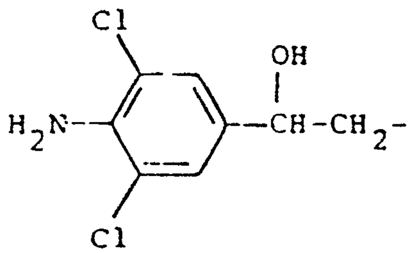
,
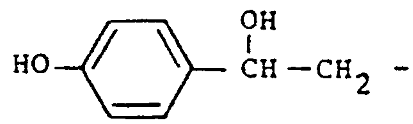

6. Composé suivant l'une quelconque des revendications 1 à 5, caractérisé en ce que $R^4$ est en position para.

7. Composé suivant l'une quelconque des revendications 1 à 6, caractérisé en ce que $R^4$ représente un radical de formule $-O(CH_2)_bCOOH$ ou un dérivé ester ou amide de celui-ci.

8. Composé suivant l'une quelconque des revendications 1 à 7, caractérisé en ce que b représente 1.

9. Composé suivant la revendication 1, caractérisé en ce qu'il est choisi dans le groupe comprenant les composés suivants :
4-[(R)-2-[bis-[(S)-2-hydroxy-3-phénoxypropyl]amino]propyl]benzoate de méthyle ;
4-[(R)-2-[bis-[(R)-2-hydroxy-3-phénoxypropyl]amino]propyl]benzoate de méthyle ;
N-méthyl 4-[(R)-2-[bis-[(S)-2-hydroxy-3-phénoxypropyl]amino]propyl]benzamide ;
4-[(R)-2-[N-[(R)-2-hydroxy-2-phénéthyl]-N-[(S)-2-hydroxy-3-phénoxypropyl]amino]propyl]benzoate de méthyle ;
4-[(R)-2-[N-[(R)-2-hydroxy-2-phénéthyl]-N-[(R)-2-hydroxy-3-phénoxypropyl]amino]propyl]benzoate de méthyle ;
N-méthyl 4-[(R)-2-[N'-[(R)-2-hydroxy-2-phénéthyl]-N'-[(S)-2-hydroxy-3-phénoxypropyl]amino]propyl]-benzamide ;
N-méthyl 4-[(R)-2-[N'-[(R)-2-hydroxy-2-phénéthyl]-N'-[(R)-2-hydroxy-3-phénoxypropyl]amino]propyl]-benzamide ;
4-[2-[N-[2-(3-chorophényl)-2-hydroxyéthyl]-N-[2-hydroxy-3-phénoxypropyl]amino]propyl]-phénoxyacétamide ;
4-[2-[N-[2-hydroxy-2-[4-hydroxy-3-hydroxyméthylphényl]éthyl]-N-[2-hydroxy-3-phénoxypropyl]amino]-propyl]benzoate de méthyle ;
4-[(R)-2-[N-[2-[4-amino-3,5-dichlorophényl]-2-hydroxyéthyl]-N-[(S)-2-hydroxy-3-phénoxypropyl]amino]-propyl]benzoate de méthyle ;
4-[2-[N-[2-(3-chlorophényl)-2-hydroxyéthyl]-N-[2-hydroxy-3-(4-carboxamidométhyl)phénoxypropyl]-amino]propyl]phénoxyacétate de émthyle ;
4-[(R)-2-[N-(R)-2-hydroxy-3-phénéthyl-N-[(S)-2-hydroxy-3-phénoxypropyl]amino]butyl]benzamide ;
4-[(R)-2-[bis-[(S)-2-hydroxy-3-phénoxypropyl]amino]propyl]phénoxyacétamide ;
4-[(R)-2-[N-[(R)-2-hydroxy-2-phénéthyl]-N-[(S)-2-hydroxy-3-phénoxypropyl]amino]propyl]phénoxyacétate de méthyle ;
4-[(R)-2-[N-[(R)-2-hydroxy-2-phénéthyl]-N-[(S)-2-hydroxy-3-phénoxypropyl]amino]propyl]-phénoxyacétamide ;
4-[(R)-2-[bis-[(S)-2-hydroxy-3-phénoxypropyl]amino]propyl]phénoxyacétate d'éthyle ;
4-[(R)-2-[N-[(R)-2-(3-chorophényl)-2-hydroxyéthyl]-N-[(S)-2-hydroxy-3-phénoxypropyl]amino]propyl]-phénoxyacétate d'éthyle ;

4-[(R)-2-[N-[-(R)-2-(3-chlorophényl)-2-hydroxyéthyl]-N-[(S)-2-hydroxy-3-phénoxypropyl]amino]propyl]-phénoxyacétamide ;
4-[(R)-2-[N-[(R)-2-hydroxy-2-phénéthyl]-N-[(S)-2-hydroxy-3-phénoxypropyl]amino]propyl]-phénoxyéthylamine ;
4-[(R)-3-[N-[(R)-2-hydroxy-2-phénéthyl]-N-[(S)-2-hydroxy-3-phénoxypropyl]amino]butyl]phénoxyacétate d'éthyle ;
4-[(R)-2-[N-[(R)-2-hydroxy-2-phénéthyl]-N-[(S)-2-hydroxy-3-phénoxypropyl]amino]propyl]phénoxyéthanol ;
4-[(R)-2-[N-[(R)-2-hydroxy-2-phénéthyl]-N-[(R)-2-hydroxy-3-phénoxypropyl]amino]propyl]-phénoxyacétate d'éthyle ;
4-[(R)-2-[N-[(R)-2-hydroxy-2-phénéthyl]-N-[(S)-2-hydroxy-3-phénoxypropyl]amino]propyl]phénoxyacétate de méthyle ;
4-[(R)-2-[N-[(R)-2-hydroxy-2-phénéthyl]-N-[(S)-2-hydroxy-3-phénoxypropyl]amino]propoxy]-phénoxyacétamide ;
4-[(R)-2-[N-[2-(2-benzofuranyl)-2-hydroxyéthyl]-N-[(S)-2-hydroxy-3-phénoxypropyl]amino]propyl]-phénoxyacétate d'éthyle ;
4-[(R)-2-[N-[(R)-2-hydroxy-2-phénéthyl]-N-[2-(2-benzofuranyl)-2-hydroxyéthyl]amino]propyl]-phénoxyacétate d'éthyle ;
4-[(R)-2-[N-[(R)-2-hydroxy-2-(4-hydroxyphényléthyl]-N-[(R)-2-hydroxy-3-phénoxypropyl]amino]propyl]-phénoxyacétamide ;
4-[2-[N-[2-(4-amino-3,5-dichlorophényl)-2-hydroxyéthyl]-N-[2-hydroxy-3-phénoxypropyl]amino]propyl]-phénoxyacétate de méthyle ; et
4-[2-[N-[2-(4-amino-3,5-dichlorophényl)-2-hydroxyéthyl]-N-[2-hydroxy-3-phénoxypropyl]amino]propyl]-phénoxyacétamide ; ou un sel de ceux-ci acceptable du point de vue pharmaceutique.

**10.** Procédé pour la préparation d'un composé de formule générale (I) telle que définie dans la revendication 1, ou d'un sel de celui-ci acceptable du point de vue pharmaceutique, caractérisé en ce que le procédé comprend :

(A) la réaction d'un composé de formule générale (II) :

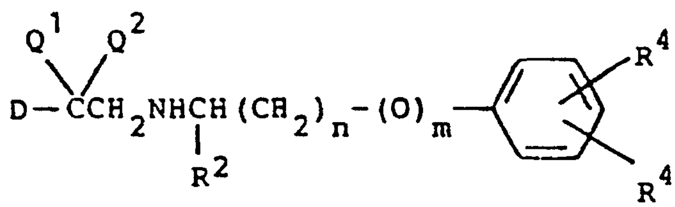

(II)

dans laquelle $R^2$, D, $R^4$, $R^{4'}$, n, m, $Q^1$ et $Q^2$ sont tels que définis à propos de la formule (I) dans la revendication 1 avec

(i) un composé de formule générale :

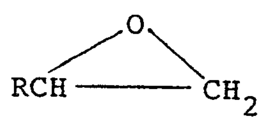

(III)

dans laquelle R représente une partie benzofuran-2-yle ou un groupe de formule générale :

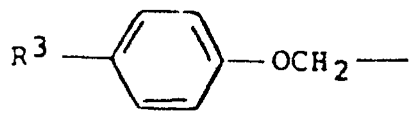

or

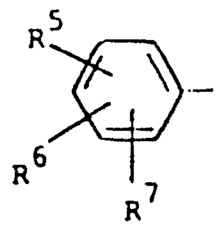

dans laquelle $R^3$, $R^5$, $R^6$ et $R^7$ sont tels que définis à propos de la formule (I) dans la revendication 1, ou

(ii) un composé de formule générale (IV) :

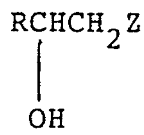

(IV)

dans laquelle R est tel que défini à propos de la formule (III) et Z représente un groupe mobile, ou un ester de celui-ci ; ou

(B) pour la préparation d'un composé de formule générale (I) dans laquelle il y a un groupe oxo représenté par $Q^1 + Q^2$, ou d'un sel de celui-ci acceptable du point de vue pharmaceutique, la réaction d'un composé de formule générale (II) telle que définie plus haut à condition que $Q^1$ et $Q^2$ ne représentent pas un groupe oxo, avec un composé de formule générale (V) :

$RCOCH_2Z$ (V)

dans laquelle R et Z sont tels que définis à propos de la formule (IV) et, si désiré, la conversion du composé résultant de formule générale (VI) :

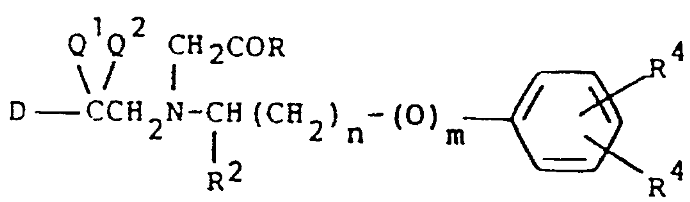

(VI)

dans laquelle R, $R^2$, D, $R^4$, $R^4$ , n, m, $Q^1$ et $Q^2$ sont tels que définis à propos de la formule (I) dans la revendication 1, et à condition que $Q^1$ et $Q^2$ ne représentent pas un groupe oxo, en un autre composé de formule générale (VI) ou (I) ; ou

(C) la réaction d'un composé de formule générale (IX) :

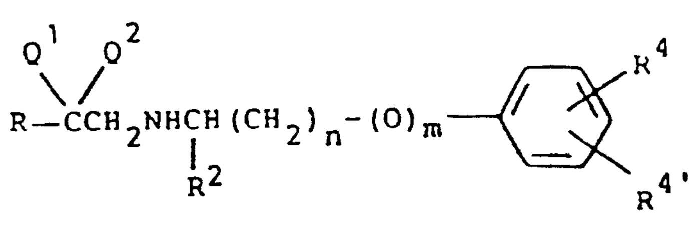

(IX)

dans laquelle $R^2$, $R^4$,$R^{4'}$, n, m, $Q^1$ et $Q^2$ sont tels que définis à propos de la formule (I) dans la revendication 1 et R est tel que défini à propos de la formule (III), avec le composé défini plus de formule générale (VII)a ou (VII)b, à condition que dans le cas ou $Q^1$ et $Q^2$ représentent ensemble un groupe carbonyle, le composé (IX) soit ensuite alors traité avec un composé (VII)a ; ou

(D) la réaction d'un composé de formule générale (II) mentionnée plus haut :

(i) avec un composé de formule générale (X) : R-CO.CHO (X)

dans laquelle R est tel que défini à propos de la formule (III) et le traitement ultérieur avec un agent réducteur,

ou (ii) avec un composé de formule générale (XI) :

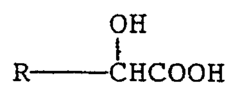

(XI)

dans laquelle R est tel que défini à propos de la formule (III) et la réduction ultérieure de l'hydroxyamide résultant ; ou

(E) la réaction d'un composé de formule générale (XII) :

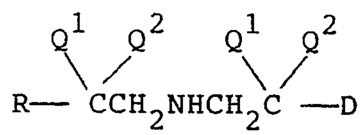

(XII)

dans laquelle D, $Q^1$ et $Q^2$ sont tels que définis à propos de la formule (I) dans la revendication 1 et R est tel que défini à propos de la formule (III) ,

(i) avec un composé de formule générale (XIII) :

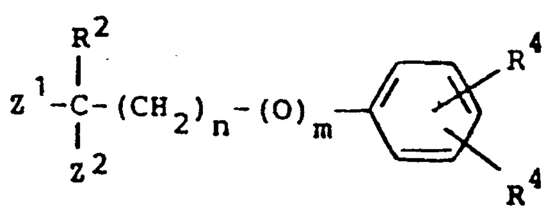

(XIII)

dans laquelle $R^2$, $R^4$, $R^{4'}$, n et m sont tels que définis à propos de la formule (I) dans la revendication 1 et $Z^1$ représente un groupe mobile et $Z^2$ représente un atome d'hydrogène, ou

(ii) avec un composé de formule générale (XIII) dans laquelle $R^2$, $R^4$, $R^{4'}$, n et m sont tels que définis à propos de la formule (I) dans la revendication 1, et $Z^1$ et $Z^2$ représentent ensemble un groupe oxo ; et si nécessaire, le traitement avec un agent réducteur ; ou

(F) la réduction d'un composé de formule générale (VI) mentionnée dans laquelle R, $R^2$, D, $R^4$, $R^4$ , n, m, $Q^1$ et $Q^2$ sont tels que définis à propos de la formule (I) dans la revendication 1 ou un d'un sel de celui-ci ;

et ensuite, si nécessaire, la conversion d'un composé de formule (I) en un autre composé de formule (I) et/ou la formation d'un sel acceptable du point de vue pharmaceutique du composé ainsi formé.

**11.** Composé de formule (I) suivant la revendication 1, ou un sel de celui-ci acceptable du point de vue pharmaceutique, caractérisé en ce qu'il est utile en tant que substance thérapeutique active.

**12.** Composé de formule (I) suivant la revendication 1 ou un sel de celui-ci acceptable du point de vue pharmaceutique, caractérisé en ce qu'il est utile dans le traitement de l'obésité et/ou l'hyperglycémie chez l'homme ou des animaux non-humains.

13. Composition pharmaceutique caractérisée en ce qu'elle comprend un composé de formule (I) suivant la revendication 1 ou un sel de celui-ci acceptable du point de vue pharmaceutique avec un support pour celui-ci acceptable du point de vue pharmaceutique.

14. Procédé pour améliorer la prise de poids et/ou augmenter le rendement de l'utilisation des aliments et/ou augmenter la masse corporelle maigre des animaux sur pieds, caractérisé en ce qu'il comprend l'administration à ces animaux sur pieds d'une quantité nontoxique efficace d'un composé de formule (I) suivant la revendication 1 ou d'un sel de celui-ci acceptable du point de vue vétérinaire.

## Ansprüche

1. Verbindung der allgemeinen Formel (I)

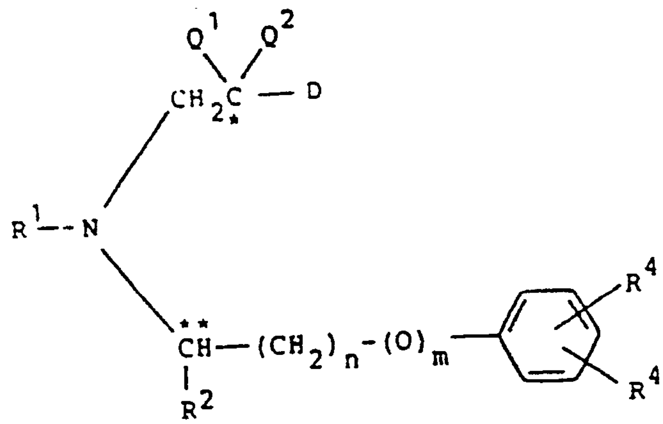

(I)

oder ein pharmazeutisch verträgliches Salz davon,
in der
$R^1$
eine 2-Hydroxy-2-(benzofuran-2-yl)-ethylgruppe oder einen Rest der allgemeinen Formel (a) oder (b)

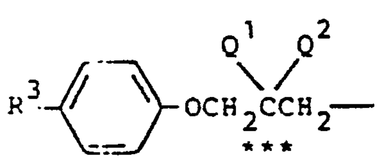

oder

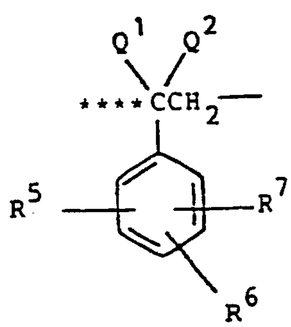

(a) (b)

bedeutet,
$R^2$
ein Wasserstoffatom oder eine Methylgruppe bedeutet,
D
entweder eine Benzofuran-2-yl-Gruppe oder einen Rest der allgemeinen Formel (c)

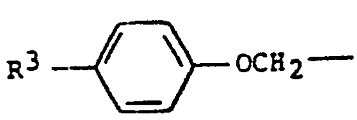

(c)

$R^3$
ein Wasserstoffatom oder eine $CH_2CONH_2$-Gruppe bedeutet und sofern in der Verbindung zwei Reste $R^3$ vorliegen, diese gleich oder verschieden sind, mit der Maßgabe, daß in der Verbindung höchstens eine $CH_2CONH_2$-Gruppe vorliegt,
$R^4$
eine der Gruppen $-(CH_2)_aCOOH$, $-O(CH_2)_bCOOH$ oder $-C(R^8)=C(R^9)COOH$ oder einen Ester oder ein Amid, ein Niederalkylamid, ein Di(niederalkyl)amid oder ein gesättigtes, aus 5- oder 6-gliedrigen Ringen bestehendes Amidderivat einer dieser Gruppen, oder einen der Reste $-SO_2NR^{10}R^{11}$, $-(CH_2)_aCH_2X$ oder $-O(CH_2)_bCH_2X$ bedeutet, wobei
a
die Zahl Null oder eine ganze Zahl von 1 bis 6 bedeutet,
b
eine ganze Zahl von 1 bis 6 bedeutet,
$R^8$ und $R^9$,
die gleich oder verschieden sein können, jeweils ein Wasserstoffatom oder eine Methylgruppe bedeuten,
$R^{10}$ und $R^{11}$,
die gleich oder verschieden sein können, jeweils ein Wasserstoffatom oder einen Niederalkylrest bedeuten, oder zusammen mit dem Stickstoffatom, an das sie gebunden sind, einen gesättigten 5- oder 6-gliedrigen Ring bilden, und
X
ein Wasserstoffatom oder einen Niederalkylrest, eine Hydroxylgruppe oder einen Ester davon, einen Niederalkoxyrest, einen unsubstituierten oder substituierten Benzyloxyrest oder einen (Niederalkyl)-carbonylrest, oder einen Rest $NR^{10}R^{11}$, wobei $R^{10}$ und $R^{11}$ die vorstehend angegebenen Bedeutungen haben, bedeutet,
$R^4$,
ein Wasserstoffatom oder ein Halogenatom oder einen Niederalkyl- oder Niederalkoxyrest, eine $CF_3$-Gruppe oder eine Hydroxylgruppe oder einen Ester davon bedeutet,
$R^5$
ein Wasserstoffatom oder ein Halogenatom, eine Hydroxy-oder Hydroxymethylgruppe oder einen Ester einer dieser Gruppen, oder einen $CF_3$-, $NH_2$-, $NHR^{12}$-, $NHCOOR^{12}$- oder $CH_2SO_2R^{12}$-Rest bedeutet, wobei $R^{12}$ einen Niederalkylrest bedeutet,
$R^6$
ein Wasserstoffatom oder ein Halogenatom oder eine Hydroxylgruppe oder einen Ester davon bedeutet,
$R^7$
ein Wasserstoffatom oder Halogenatom bedeutet,
n
die Zahl 1 oder 2 bedeutet,
m
die Zahl 0 oder 1 bedeutet, mit der Maßgabe, daß $n + m$ 1 oder 2 ist, und
$Q^1$
ein Wasserstoffatom bedeutet und
$Q^2$
eine Hydroxylgruppe oder einen pharmazeutisch verträglichen in vivo hydrolysierbaren Ester davon bedeutet, oder
$Q^1$ und $Q^2$
zusammen eine Oxogruppe bedeuten, wobei die zwei $Q^1Q^2$-Paare in der Verbindung gleich oder verschieden sind, mit der Maßgabe, daß höchstens eine $Q^1Q^2$-Oxogruppe in der Verbindung vorliegt;
mit der folgenden Maßgabe: Sofern $R^1$ einen Rest der vorstehend erwähnten Formel (a) bedeutet, in der $R^3$ und $Q^1$ jeweils ein Wasserstoffatom und $Q^2$ eine Hydroxylgruppe oder einen pharmazeutisch verträglichen in vivo hydrolysierbaren Ester davon bedeutet, oder $R^1$ einen Rest der vorstehend erwähnten Formel (b) bedeutet, in der $R^5$ ein Wasserstoffatom oder ein Halogenatom oder eine Trifluormethylgruppe und $R^6$, $R^7$ und $Q^1$ jeweils ein Wasserstoffatom und $Q^2$ eine Hydroxylgruppe oder einen in vivo hydrolysierbaren Ester davon bedeutet; und $R^2$ ein Wasserstoffatom oder eine Methylgruppe bedeutet; und
D einen Rest der vorstehend erwähnten Formel (c) bedeutet, in der $R^3$ ein Wasserstoffatom bedeutet; $Q^1$ ein Wasserstoffatom und $Q^2$ eine Hydroxylgruppe oder einen in vivo hydrolysierbaren Ester davon bedeutet;

$R^4$ einen Substituenten am Phenylring in para-Stellung zur $-(O)_m$-Gruppe bedeutet, und entweder ein Rest $-(CH_2)_aCO_2H$, $-C(R^8)=C(R^9)CO_2H$, oder einen Ester oder ein Amid, ein Niederalkylamid oder ein Di(niederalkyl)amid einer dieser Gruppen bedeutet, oder einen der Reste $-SO_2NR^{10}R^{11}$, $-(CH_2)_aCH_2X$ darstellt, wobei X eine Hydroxylgruppe oder einen (Niederalkyl)carbonylrest bedeutet,
oder
einen $-O(CH_2)_bCH_2X$-Rest bedeutet, wobei X eine Hydroxylgruppe, einen Niederalkoxyrest oder eine unsubstituierte Benzoyloxygruppe bedeutet,
a die Zahl 0 oder eine ganze Zahl von 1 bis 6 ist,
b eine ganze Zahl von 1 bis 6 ist,
$R^8$ ein Wasserstoffatom oder eine Methylgruppe bedeutet,
$R^9$, $R^{10}$ und $R^{11}$ jeweils ein Wasserstoffatom bedeutet,
n die Zahl 1 oder 2 ist und m 0 ist;
dann ist $R^{4'}$, kein Wasserstoffatom.

2. Verbindung nach Anspruch 1, in der jeder der Reste (a) und (b), die für $R^1$ und $Q^1$ stehen ein Wasserstoffatom bedeutet, und $Q^2$ eine Hydroxylgruppe oder einen pharmazeutisch verträglichen in vivo hydrolysierbaren Ester davon bedeutet.

3. Verbindung nach Anspruch 1 oder 2 mit der Formel (I')

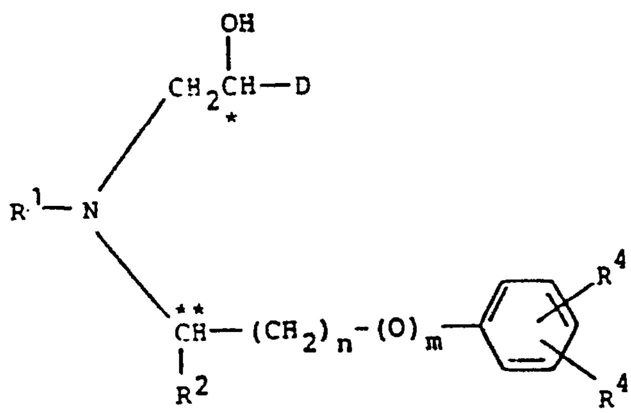

(I')

oder ein pharmazeutisch verträgliches Salz davon, in der $R^1$ eine 2-Hydroxy-2-(benzofuran-2-yl)-ethylgruppe oder einen Rest der allgemeinen Formel (a') oder (b') bedeutet,

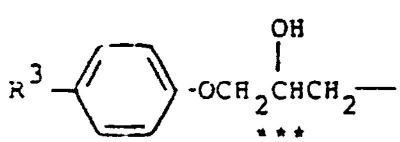

oder

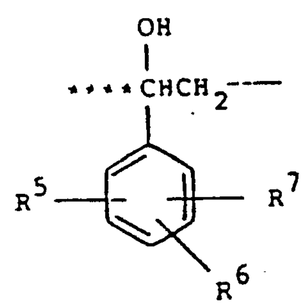

(a′) (b′)

die Reste $R^2$, $R^3$, $R^4$, $R^{4'}$, $R^5$, $R^6$, $R^7$, m und n gemäß der Formel (I) in Anspruch 1 definiert sind, und D eine Benzofuran-2-yl-Gruppe oder einen Rest der Formel (c) wie in Anspruch 1 definiert, bedeutet.

4. Verbindung nach einem der Ansprüche 1 bis 3, in der $R^1$ eine 2-Hydroxy-2-(benzofuran-2-yl)ethyl-Gruppe oder eine Gruppe der Formel (a') oder (b') bedeutet

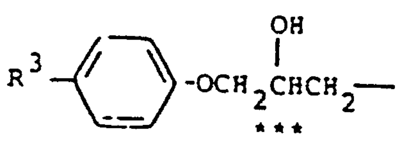

oder

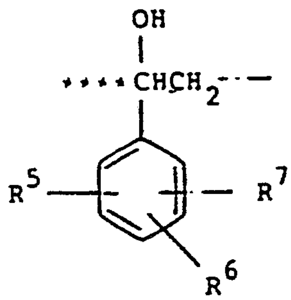

(a')

(b')

die Reste $R^3$, $R^6$ und $R^7$, gemäß der Formel (I) in Anspruch 1 definiert sind, und
$R^5$ ein Wasserstoffatom oder ein Halogenatom, eine Hydroxy-oder Hydroxymethylgruppe oder einen Ester einer dieser Gruppen oder eine $NH_2$- oder $NHR^{12}$-Gruppe bedeutet, wobei
$R^{12}$ einen Niederalkylrest darstellt.

5. Verbindung nach einem der Ansprüche 1 bis 4, in der die Gruppe der Formel (b) eine der folgenden Gruppen bedeutet:

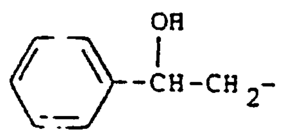

,

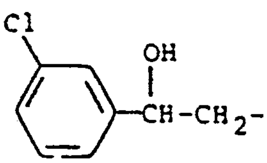

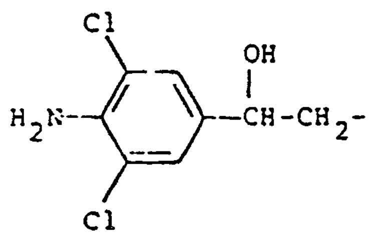

,

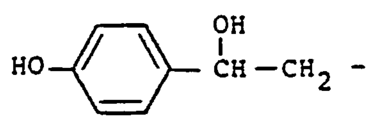

6. Verbindung nach einem der Ansprüche 1 bis 5, in der $R^4$ in para-Stellung ist.

7. Verbindung nach einem der Ansprüche 1 bis 6, in der $R^4$ einen Rest der Formel $-O(CH_2)_bCOOH$ oder ein Ester- oder Amidderivat davon bedeutet.

8. Verbindung nach einem der Ansprüche 1 bis 7, in der b die Zahl 1 ist.

9. Verbindung nach Anspruch 1, ausgewählt aus der Gruppe:

4-[(R)-2-[Bis-[(S)-2-hydroxy-3-phenoxypropyl]-amino]propyl]-benzoesäure-methylester,

4-[(R)-2-[Bis-[(R)-2-hydroxy-3-phenoxypropyl]-amino]propyl]-benzoesäure-methylester,

N-Methyl-4-[(R)-2-[bis-[(S)-2-hydroxy-3-phenoxypropyl]amino]propyl]-benzamid,

4-[(R)-2-[N-[(R)-2-Hydroxy-2-phenethyl]-N-[(S)-2-hydroxy-3-phenoxypropyl]amino]propyl]-benzoesäure-methylester,

4-[(R)-2-[N-[(R)-2-Hydroxy-2-phenethyl]-N-[(R)-2-hydroxy-3-phenoxypropyl]amino]propyl]-benzoesäure-methylester,

N-Methyl-4-[(R)-2-[N'-[(R)-2-hydroxy-2-phenethyl]-N'-[(S)-2-hydroxy-3-phenoxypropyl]amino]propyl]-benzamid,

N-Methyl-4-[(R)-2-[N'-[(R)-2-hydroxy-2-phenethyl]-N'-[(R)-2-hydroxy-3-phenoxypropyl]amino]propyl]-benzamid,

4-[2-[N-[2-(3-Chlorphenyl)-2-hydroxyethyl]-N-[2-hydroxy-3-phenoxypropyl]amino]propyl]phenoxy-acetamid,

4-[2-[N-[2-Hydroxy-2-[4-hydroxy-3-hydroxymethylphenyl]ethyl]-N-[2-hydroxy-3-phenoxypropyl]amino]-propyl]benzoesäure-methylester,

4-[(R)-2-[N-[2-[4-Amino-3,5-dichlorphenyl]-2-hydroxyethyl]-N-[(S)-2-hydroxy-3-phenoxypropyl]amino]-propyl]benzoesäure-methylester,

4-[2-[N-[2-(3-Chlorphenyl)-2-hydroxyethyl]-N-[2-hydroxy-3-(4-carboxamidomethyl)phenoxypropyl]-amino]propyl]-phenoxyessigsäure-methylester,

4-[(R)-3-[N-(R)-2-Hydroxy-2-phenethyl-N-[(S)-2-hydroxy-3-phenoxypropyl]amino]butyl]benzamid,

4-[(R)-2-[Bis-(S)-2-hydroxy-3-phenoxypropyl]amino]propyl]phenoxyacetamid,

4-[(R)-2-[N-[(R)-2-Hydroxy-2-phenethyl]-N-[(S)-2-hydroxy-3-phenoxypropyl]amino]propyl]-phenoxyessigsäure-ethylester,

4-[(R)-2-[N-[(R)-2-Hydroxy-2-phenethyl]-N-[(S)-2-hydroxy-3-phenoxypropyl]amino]propyl]-phenoxyacetamid,

4-[(R)-2-Bis-[(S)-2-hydroxy-3-phenoxypropyl]amino]propyl]-phenoxyessigsäure-ethylester,

4-[(R)-2-[N-[(R)-2-(3-Chlorphenyl)-2-hydroxyethyl]-N-[(S)-2-hydroxy-3-phenoxypropyl]amino]propyl]-phenoxyessigsäure-ethylester,

4-[(R)-2-[N-[(R)-2-(3-Chlorphenyl)-2-hydroxyethyl]-N-[(S)-2-hydroxy-3-phenoxypropyl]amino]propyl]-phenoxyacetamid

4-[(R)-2-[N-[(R)-2-Hydroxy-2-phenethyl]-N-[(S)-2-hydroxy-3-phenoxypropyl]amino]propyl]-phenoxy-ethylamin,

4-[(R)-3-[N-[(R)-2-Hydroxy-2-phenethyl]-N-[(S)-2-hydroxy-3-phenoxypropyl]amino]butyl]-phenoxyessigsäure-ethylester,

4-[(R)-2-[N-[(R)-2-Hydroxy-2-phenethyl]-N-[(S)-2-hydroxy-3-phenoxypropyl]amino]propyl]-phenoxyethanol,

4-[(R)-2-[N-[(R)-2-Hydroxy-2-phenethyl]-N-[(R)-2-hydroxy-3-phenoxypropyl]amino]propyl]-phenoxyessigsäure-ethylester,

4-[(R)-2-[N-[(R)-2-Hydroxy-2-phenethyl]-N-[(S)-2-hydroxy-3-phenoxypropyl]amino]propyl]-phenoxyessigsäure-methylester,

4-[(R)-2-[N-[(R)-2-Hydroxy-2-phenethyl]-N-[(S)-2-hydroxy-3-phenoxypropyl]amino]propoxy]-phenoxyacetamid,

4-[(R)-2-[N-[2-(2-Benzofuranyl)-2-hydroxyethyl-N-[(S)-2-hydroxy-3-phenoxypropyl]amino]propyl]-phenoxyessigsäure-ethylester,

4-[(R)-2-[N-[(R)-2-Hydroxy-2-phenethyl]-N-[2-(2-benzofuranyl)-2-hydroxyethyl]amino]propyl]-phenoxyessigsäure-ethylester,

4-[(R)-2-[N-[(R)-2-Hydroxy-2-(4-hydroxyphenylethyl]-N-[(R)-2-hydroxy-3-phenoxypropyl]amino]propyl]-phenoxyacetamid,

4-[2-[N-[2-(4-Amino-3,5-dichlorphenyl)-2-hydroxyethyl]-N-[2-hydroxy-3-phenoxypropyl]amino]propyl]-phenoxyessigsäure-methylester und

4-[2-[N-[2-(4-Amino-3,5-dichlorphenyl)-2-hydroxyethyl]-N-[2-hydroxy-3-phenoxypropyl]amino]propyl]-phenoxyacetamid oder ein pharmazeutisch verträgliches Salz davon.

**10.** Verfahren zur Herstellung einer Verbindung der allgemeinen Formel (I) wie in Anspruch 1 definiert, oder eines pharmazeutisch verträglichen Salzes davon, dadurch gekennzeichnet, daß das Verfahren die folgenden Stufen umfaßt:

A) Umsetzen einer Verbindung der allgemeinen Formel (II)

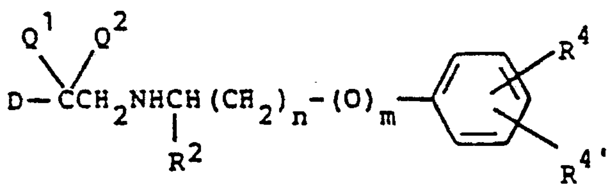

(II)

in der $R^2$, D, $R^4$, $R^{4'}$, n, m, $Q^1$ und $Q^2$ gemäß Formel (I) in Anspruch 1 definiert sind,

(i) mit einer Verbindung der allgemeinen Formel

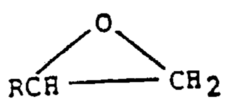

(III)

in der R eine Benzofuran-2-yl-Gruppe oder eine Gruppe der nachstehenden allgemeinen Formeln ist

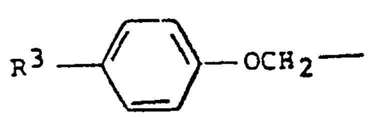

oder

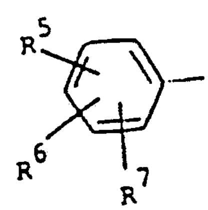

in der die Reste $R^3$, $R^5$, $R^6$ und $R^7$ gemäß Formel (I) in Anspruch 1 definiert sind, oder
(ii) mit einer Verbindung der allgemeinen Formel (IV)

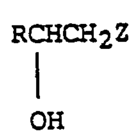

(IV)

in der R gemäß Formel (III) definiert ist und Z eine Abgangsgruppe oder einen Ester davon

bedeutet;
oder

B) zur Herstellung einer Verbindung der allgemeinen Formel (I), in der eine Oxogruppe dargestellt durch $Q^1 + Q^2$ vorhanden ist, oder eines pharmazeutisch verträglichen Salzes davon, durch Umsetzen einer Verbindung der allgemeinen Formel (II), wie vorstehend definiert, mit der Maßgabe, daß $Q^1$ und $Q^2$ nicht eine Oxogruppe bedeuten, mit einer Verbindung der allgemeinen Formel (V)

$RCOCH_2Z$ (V)

in der R und Z gemäß Formel (III) definiert sind und, falls gewünscht, durch Umwandeln der entstandenen Verbindung der allgemeinen Formel (VI):

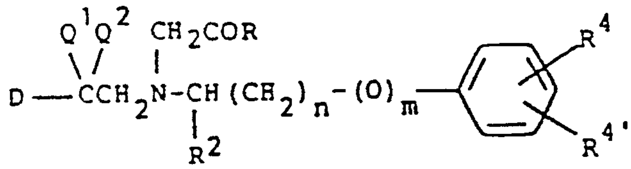

(VI)

in der R, $R^2$, D, $R^4$, $R^{4'}$, n, m, $Q^1$ und $Q^2$ gemäß Formel (I) in Anspruch 1 definiert sind und mit der Maßgabe, daß $Q^1$ und $Q^2$ nicht eine Oxogruppe bedeuten, in eine andere Verbindung der allgemeinen Formel (VI) (oder I), oder

C) durch Umsetzen einer Verbindung der allgemeinen Formel (IX)

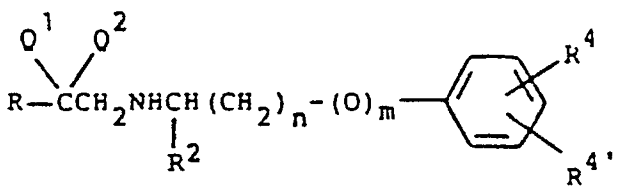

(IX)

in der $R^2$, $R^4$, $R^{4'}$, n, m, $Q^1$ und $Q^2$ gemäß Formel (I) in Anspruch 1 definiert sind und R gemäß Formel (III) definiert ist, mit der vorstehend definierten Verbindung der allgemeinen Formel (VII)a oder (VII)b, mit der Maßgabe, daß, falls $Q^1$ und $O^2$ zusammen eine Carbonylgruppe bedeuten, dann die Verbindung (IX) mit der Verbindung (VII)a umgesetzt wird;
oder

D) durch Umsetzen einer Verbindung der vorstehend erwähnten allgemeinen Formel (II)

(i) mit einer Verbindung der allgemeinen Formel (X):

R-CO.CHO (X)

in der R gemäß Formel (III) definiert ist, und nachfolgendes Behandeln mit einem Reduktionsmittel, oder

(ii) mit einer Verbindung der allgemeinen Formel (XI)

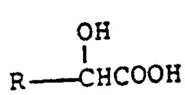

(XI)

in der R gemäß Formel (III) definiert ist und nachfolgend Reduzieren des entstandenen Hydroxylamids, oder

E) durch Umsetzen einer Verbindung der allgemeinen Formel (XII)

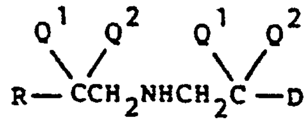

(XII)

in der D, $Q^1$ und $Q^2$ gemäß Formel (I) in Anspruch 1 definiert sind und R gemäß Formel (III) definiert ist,

(i) mit einer Verbindung der allgemeinen Formel (XIII)

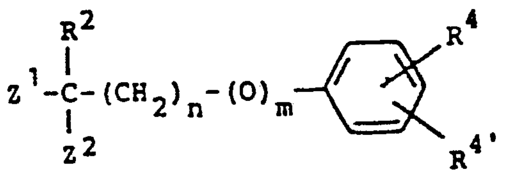

(XIII)

in der $R^2$, $R^4$, $R^{4'}$, n und m gemäß Formel (I) in Anspruch 1 definiert sind, $Z^1$ eine Abgangsgruppe und $Z^2$ ein Wasserstoffatom bedeutet, oder

(ii) mit einer Verbindung der allgemeinen Formel (XIII), in der $R^2$, $R^4$, $R^{4'}$, n und m, gemäß Formel (I) in Anspruch 1 definiert sind und $Z^1$ und $Z^2$ zusammen eine Oxogruppe bedeuten; und gegebenenfalls Behandeln mit einem Reduktionsmittel; oder

F) durch Reduktion einer Verbindung der vorstehend erwähnten allgemeinen Formel (VI), in der R, $R^2$, D, $R^4$, $R^{4'}$, n, m, $Q^1$ und $Q^2$ gemäß Formel (I) in Anspruch 1 definiert sind, oder eines Salzes davon, und danach gegebenenfalls Umwandeln einer Verbindung der Formel (I) in eine andere Verbindung der Formel (I) und/oder Bilden eines pharmazeutisch verträglichen Salzes der so gebildeten Verbindung.

**11.** Verbindung der Formel (I) gemäß Anspruch 1, oder ein pharmazeutisch verträgliches Salz davon, zur Verwendung als eine therapeutisch wirksame Substanz.

**12.** Verbindung der Formel (I) gemäß Anspruch 1, oder ein pharmazeutisch verträgliches Salz davon, zur Verwendung bei der Behandlung von Fettleibigkeit und/oder Hyperglykämie bei Menschen oder nichtmenschlichen Tierarten.

**13.** Arzneimittel, enthaltend eine Verbindung der Formel (I) gemäß Anspruch 1, oder ein pharmazeutisch verträgliches Salz davon, mit einem hierfür geeigneten pharmazeutisch verträglichen Träger.

**14.** Verfahren zur Verbesserung der Gewichtszunahme und/oder Verbesserung des Futterverwertungsgrades und/oder Erhöhung der mageren Körpermasse von Vieh, umfassend das Verabreichen einer wirksamen, ungiftigen Menge einer wie in Anspruch 1 definierten Verbindung der Formel (I) oder ein tiermedizinisch verträgliches Salz davon an das Vieh.